# EUROPEAN PATENT SPECIFICATION

(11) **EP 3 813 884 B1**
(45) Date of publication and mention of the grant of the patent: **18.09.2024**
(21) Application number: 19827332.8
(22) Date of filing: 28.06.2019
(51) Int. Cl.: A61K 9/06, A61K 9/51, A61K 9/70, A61K 47/42, A61K 39/39, A61P 35/00, A61P 35/04, C07K 16/28

(54) **IN SITU SPRAYED BIORESPONSIVE IMMUNOTHERAPEUTIC GEL FOR POST-SURGICAL TREATMENT**
IN SITU GESPRÜHTES BIORESPONSIVES IMMUNTHERAPEUTISCHES GEL ZUR POSTOPERATIVEN BEHANDLUNG
GEL IMMUNOTHÉRAPEUTIQUE BIOSENSIBLE PULVÉRISÉ IN SITU POUR UN TRAITEMENT POST-CHIRURGICAL

(30) Priority: 29.06.2018 US 201862692526 P
(43) Date of publication of application: 05.05.2021
(73) Proprietor: North Carolina State University, Raleigh, North Carolina 27606 (US)
(72) Inventor: GU, Zhen, Los Angeles, CA 90049 (US); CHEN, Qian, Raleigh, North Carolina 27606 (US)
(74) Representative: Berggren Oy
(86) International application number: PCT/US2019/039951
(87) International publication number: WO 2020/006493

(56) References cited:
- US-A- 6 074 837
- US-A1- 2018 086 807
- YATA TOMOYA ET AL: "DNA nanotechnology-based composite-type gold nanoparticle-immunostimulatory DNA hydrogel for tumor photothermal immunotherapy", BIOMATERIALS, vol. 146, 1 November 2017 (2017-11-01), AMSTERDAM, NL, pages 136 - 145, XP055891315, ISSN: 0142-9612, Retrieved from the Internet <URL:https://www.sciencedirect.com/science/article/pii/S014296121730577X/pdfft?md5=685f0d652aa8e0fb2d3d096bc75eb74f&pid=1-s2.0-S014296121730577X-main.pdf> DOI: 10.1016/j.biomaterials.2017.09.014
- ZHANG HONGBO ET AL: "Gold Nanorods Conjugated Porous Silicon Nanoparticles Encapsulated in Calcium Alginate Nano Hydrogels Using Microemulsion Templates", NANO LETTERS, vol. 18, no. 2, 14 February 2018 (2018-02-14), US, pages 1448 - 1453, XP055891310, ISSN: 1530-6984, DOI: 10.1021/acs.nanolett.7b05210
- ZHAO JIULONG ET AL: "Design of Phase-Changeable and Injectable Alginate Hydrogel for Imaging-Guided Tumor Hyperthermia and Chemotherapy", APPLIED MATERIALS & INTERFACES, vol. 10, no. 4, 31 January 2018 (2018-01-31), US, pages 3392 - 3404, XP055891603, ISSN: 1944-8244, DOI: 10.1021/acsami.7b17608
- PARK ET AL.: "Extended release of perioperative immunotherapy prevents tumor recurrence and eliminates metastases", SCIENCE TRANSLATIONAL MEDICINE, vol. 10, no. 433, 21 March 2018 (2018-03-21), XP055579366, DOI: 10.1126/scitranslmed.aar1916
- WANG ET AL.: "In situ formed reactive oxygen species-responsive scaffold with gemcitabine and checkpoint inhibitor for combination therapy", SCIENCE TRANSLATIONAL MEDICINE, vol. 10, no. 429, 21 February 2018 (2018-02-21), XP055648279
- CHEN ET AL.: "In situ sprayed bioresponsive immunotherapeutic gel for post-surgical cancer treatment", NATURE NANOTECHNOLOGY, 10 December 2018 (2018-12-10), XP036667512

## Description

### I. BACKGROUND

1. Despite continual improvements in surgical techniques, residual microtumors and/or circulating tumor cells (CTCs) after tumor resection remain challenging. It has also been validated that the perioperative inflammation induced by trauma poses a high risk of the development of tumor reoccurrence, acceleration of local remaining tumor relapse, as well as the promotion of tumor invasion and metastasis. However, current approaches relying on established treatments, including systemic chemotherapy and radiotherapy, can carry high toxicity profiles. What are needed are new approaches to reduce tumor invasion and metastasis while avoiding issued of toxicity.

Yata Tomoya et al. (Biomaterials, voi. 146, 1 November 2017, pp. 136-145) discloses the preparation of a composite-type immunostimulatory DNA hydrogel consisting of a hexapod-like structured DNA with CpG sequences and gold nanoparticles. Zhang Hongbo et al. (Nano Letters, vol. 18, no. 2, 14 February 2018, pages 1448-1453) discloses the preparation of porous silicon nanoparticles and gold nanorods which are encapsulated in a hydrogel shell as biocompatible functional nanocarriers for the sustained release of therapeutics. Zhao Jiulong et al. (Applied Materials & Interfaces, vol. 10, no. 4, 31 January 2018, pages 3392-3404) discloses the preparation of an injectable alginate hydrogel matrix comprising nanosheets and Doxorubicin for the localized treatment of tumours.

### II. SUMMARY

2. Disclosed are methods and compositions related to immunotherapeutic bioresponsive gel matrixes and methods of using the same to treat, prevent, and/or inhibit tumor proliferation. The aspects of the invention are depicted in the independent claims.

3. In one aspect, disclosed herein are immunotherapeutic bioresponsive gel matrixes comprising a first component (for example, a H+ scavenger such as, for example, fibrinin) and a second component (such as, for example thrombin); wherein gelation of the matrix occurs when the first component comes into contact with the second component; and wherein at least one of the components (i.e., the first component and/or second component) comprises at least one first nanoparticle; wherein the at least one first nanoparticle comprises at least one anti-cancer agent, blockade inhibitor, or immunomodulatory agent.

4. Also disclosed herein are immunotherapeutic bioresponsive gel matrixes of any preceding aspect, wherein the anti-cancer agent, blockade inhibitor, or immunomodulatory agent is an anti-CD47 antibody.

5. In one aspect, disclosed herein are immunotherapeutic bioresponsive gel matrixes of any preceding aspect, wherein the immunotherapeutic bioresponsive gel matrix comprises at least one second nanoparticle and/or at least one second anti-cancer agent, blockade inhibitor, or immunomodulatory agent.

6. Also disclosed herein are methods of treating, reducing, inhibiting, and/or preventing tumor proliferation and/or metastasis at the site of surgical resection of a cancer tumor comprising administering to a subject at the site of the resection, an immunotherapeutic bioresponsive gel matrix comprising a first component and a second component; wherein gelation of the matrix occurs when the first component comes into contact with the second component; and wherein at least one of the components (i.e., the first component and/or second component) comprises at least one first nanoparticle; wherein the at least one first nanoparticle comprises at least one anti-cancer agent, blockade inhibitor, or immunomodulatory agent. Thus, in one aspect, disclosed herein are methods of treating, inhibiting, and/or preventing tumor proliferation and/or metastasis at the site of surgical resection of a cancer tumor comprising administering to a subject at the site of the resection the immunotherapeutic bioresponsive gel matrix of any preceding aspect.

7. In one aspect, disclosed herein are methods of treating, reducing, inhibiting, and/or preventing tumor proliferation and/or metastasis of any preceding aspect, wherein the first and second component are mixed prior to delivery to the subject (thereby forming the matrix prior to administration), administered and mixed simultaneously as the components are administered to the subject, or delivered to the subject separately (such as, for example, concurrent or sequential administration).

8. Also disclosed herein are methods of increasing the innate or adaptive immune response to a tumor following resection of the tumor comprising administering to a subject with a tumor an immunotherapeutic bioresponsive gel matrix comprising a first component and a second component; wherein gelation of the matrix occurs when the first component comes into contact with the second component; and wherein at least one of the components (i.e., the first component and/or second component) comprises at least one first nanoparticle; wherein the at least one first nanoparticle comprises at least one anti-cancer agent, blockade inhibitor, or immunomodulatory agent.

### III. BRIEF DESCRIPTION OF THE DRAWINGS

9. The accompanying drawings, which are incorporated in and constitute a part of this specification, illustrate several embodiments and together with the description illustrate the disclosed compositions and methods.

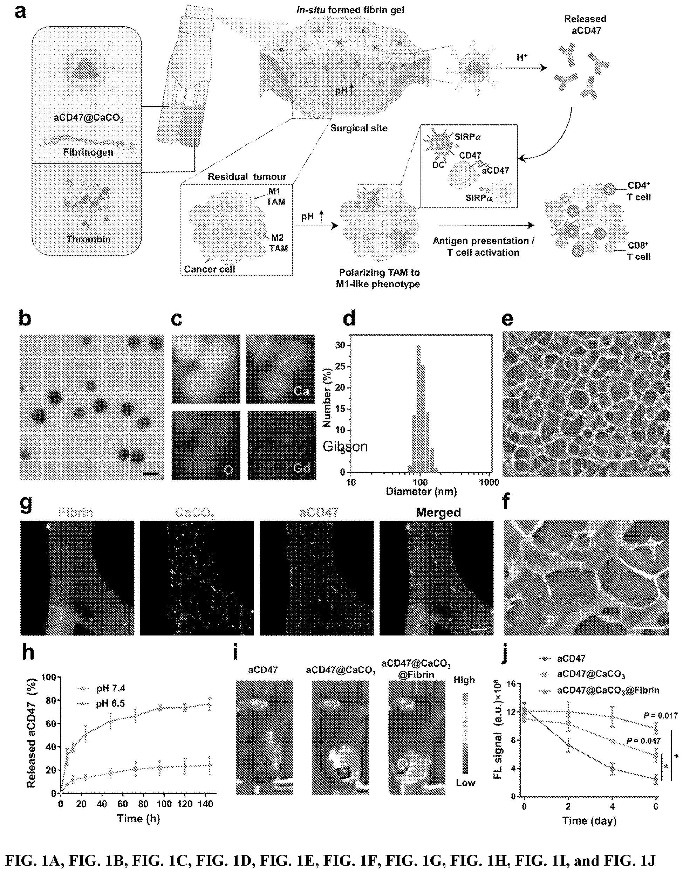

10. Figure 1A, 1B, 1C, 1D, 1E, 1F, 1G, 1H, 1I, and 1J show schematic and characterization of in situ formed immunotherapeutic fibrin gel. Figure 1A shows a schematic illustration showing the immunotherapeutic bioresponsive fibrin gel containing aCD47@CaCO3 NPs, formed in situ after being quickly sprayed, for effectively inhibiting tumor recurrence and metastasis after surgery. aCD47@CaCO3 NPs encapsulated in fibrin are able to scavenge H+ in the surgical wound site and the residual TME and subsequently release aCD47, thus eliciting immunosupportive post-resection environment, such as polarization of TAMs to an M1-like phenotype, and blocking the "don't eat me" signal expressed on cancer cells to increase the phagocytosis of cancer cells by macrophages and initiate T-cell mediated antitumor responses. Figure 1B shows TEM image of aCD47@CaCO₃ NPs (scale bar: 100 nm). The experiments were repeated thrice. Figure 1C shows Scanning TEM (STEM) images of aCD47@CaCO₃ NPs showing the calcium (green), oxygen (red), and gadolinium labeled aCD47 (green). The experiments were repeated thrice. Figure 1D shows the average hydrodynamic size of aCD47@CaCO₃ NPs determined by DLS. Figures 1E and 1F shows representative Cryo-SEM images of fibrin gel loaded with aCD47@CaCO3 NPs. Scale bar, 1 µm. Figure 1G shows representative fluorescent images of cryosection of fibrin, in which fibrinogen was labeled with FITC, CaCO3 NPs were labeled with Cy5.5, and aCD47 was stained with Rhodamine conjugated anti-rat IgG antibody. Scale bar, 10 µm. Figure 1H shows cumulative release profiles of aCD47 from fibrin in solutions with different pH values. Figure 1I shows fluorescence IVIS imaging depicting the in vivo release profile of aCD47-Cy5.5 six days after different delivery of antibodies. The experiments were repeated thrice. Figure 1J shows quantification of the in vivo retention profile (also known as a release profile) of aCD47-Cy5.5. The error bars represent standard error of the mean (s.e.m.). P value: *P < 0.05.

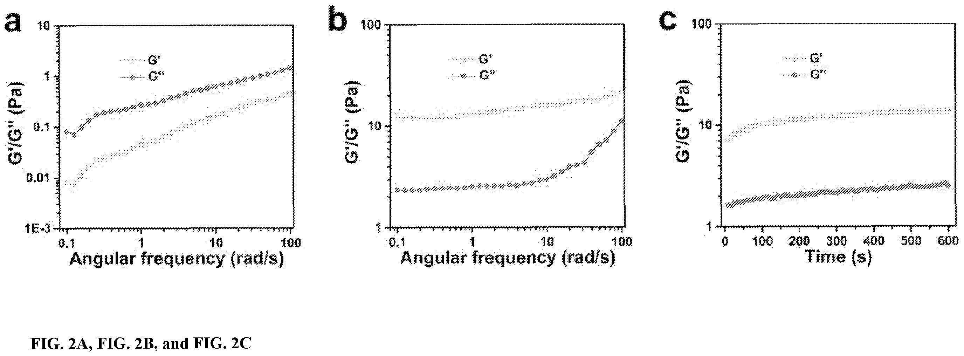

11. Figures 2A, 2B, and 2C show the dynamic rheological behavior of fibrinogen before and after gelation at 25 °C measured using a TA Instruments AR-2000 stress controlled rheometer with 25 mm aluminum cross-hatched parallel plates. All experiments were conducted in the linear viscoelastic regime with a 500 µm gap between the plates and conducted at least triplicate measurements. Figure 2A shows the frequency spectra of the elastic (G') and viscous (G") moduli of fibrinogen containing IgG@CaCO3 NPs samples with the exhibiting solution-like characteristics. Figure 2B shows the frequency spectra of the elastic (G') and viscous (G") moduli of IgG@CaCO3@Fibrin samples showing a gel-like behavior. Figure 2C shows the evolution of G' and G" as a function of time of the mixture of fibrinogen containing IgG@CaCO3 and thrombin showing ultrafast sol-gel transition. IgG (Immunoglobulin G) indicates the aCD47 used in this study.

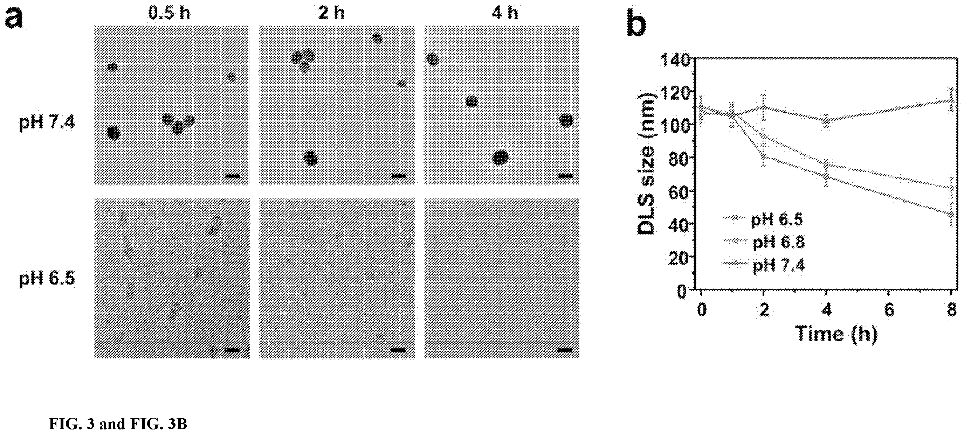

12. Figures 3A and 3B show TEM images and release profile of aCD47@CaCO3 NPs. Figure 3A shows representative TEM images of aCD47@CaCO3 NPs after being immersed in phosphate buffered saline (PBS) with different pH values (7.4 and 6.5) and for different periods of time (scale bar: 100 nm). The experiments were repeated three times. Figure 3B shows time-dependent DLS-measured size changes of aCD47@CaCO3 NPs dispersed in PBS with different pH values (6.5, 6.8, and 7.4). Data are presented as mean ± s.e.m. (n=3).

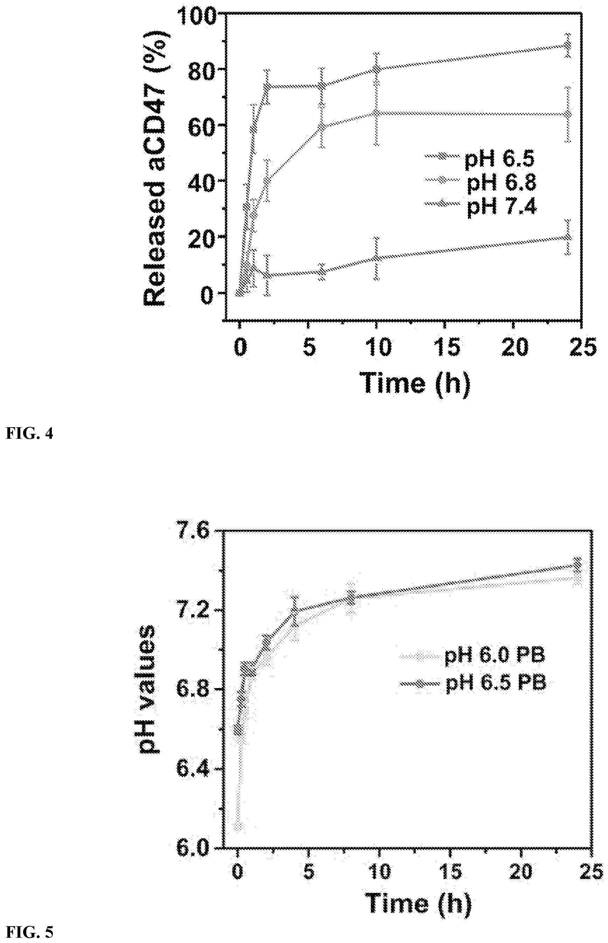

13. Figure 4 shows time-dependent DLS-measured size changes of aCD47 from CaCO3 NPs dispersed in PBS with the different pH values (6.5, 6.8, and 7.4). The error bars represent standard error of the mean (s.e.m.) (n = 3).

14. Figure 5 shows Time-dependent pH value changes of phosphate buffer (PB) containing CaCO3 NPs. The error bars represent standard error of the mean (s.e.m.) (n = 3).

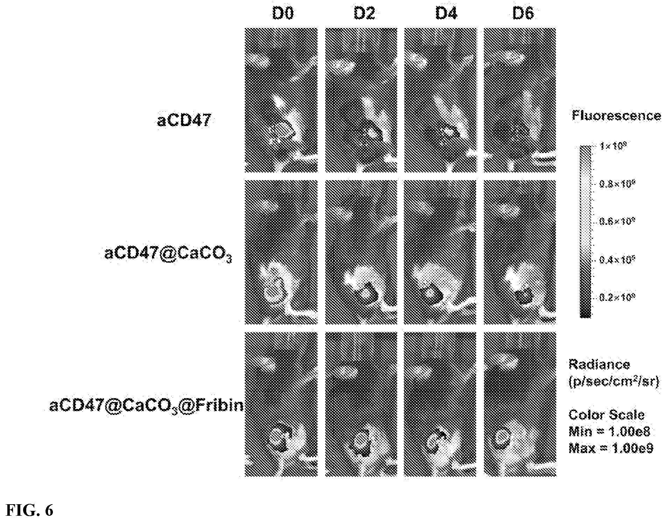

15. Figure 6 shows fluorescence IVIS imaging depicting the in vivo release profile of aCD47-Cy5.5 on different days.

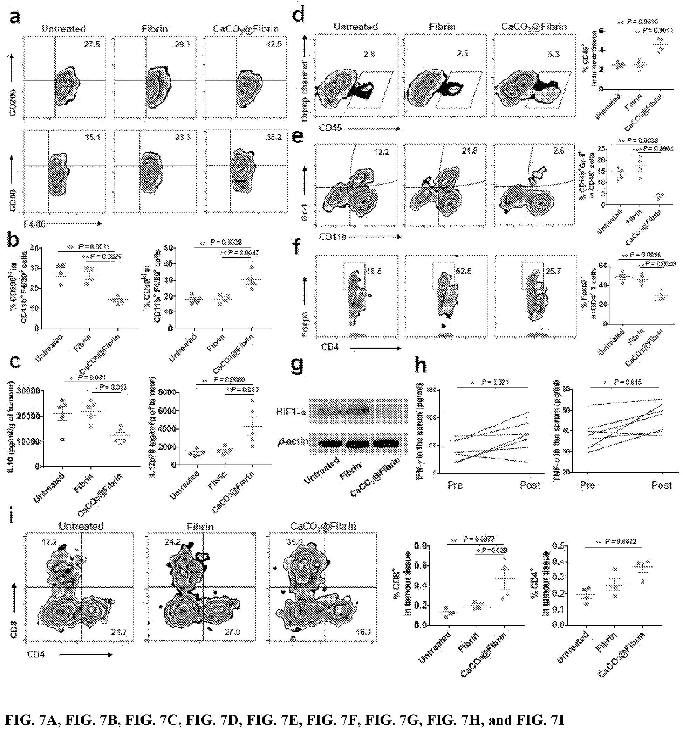

16. Figures 7A, 7B, 7C, 7D, 7E, 7F, 7G, 7H and 7I show CaCO3@ Fibrin incorporation for relieving immunosuppressive TME. B16F10 tumors were collected from mice five days after different treatments for analysis. Figure 7A shows representative flow cytometric analysis images and (7b) corresponding quantification of M2- macrophages (CD206hi) and M1-macrophages (CD80 hi) in F4/80+CD11b+ CD45+ cells. Figure 7C shows secretion levels of IL 10 and IL12p70 in different tumors. Figure 7D shows representative flow cytometric analysis images (left) and the corresponding quantification (right) of CD45+ cells. Figure 7E shows representative flow cytometric analysis images (left) and the corresponding quantification (right) of MDSCs (CD11b+Gr-1+) gating on CD45+ cells. Figure 7F shows epresentative flow cytometric analysis images (left) and the corresponding quantification (right) of CD4+Foxp3+ T cells gating on CD3+ cells. Figure 7G shows HIF1-α protein expression levels in B16F10 tumors analyzed by Western blotting. Figure 7H shows systemic IFN-γ and TNF-α levels before and after CaCO3@Fibrin treatment. Figure 7I shows epresentative flow cytometric analysis (left) and the corresponding quantification (right) of CD4+ and CD8+ T cells gating on CD3+ cells. The error bars represent standard error of the mean (s.e.m.). Statistical significance was calculated via one-way ANOVA with a Tukey post-hoc test (7b, 7c, 7d-77f, i), or Welch's t-test (7h). P value: *P < 0.05; **P < 0.01; ***P < 0.001.

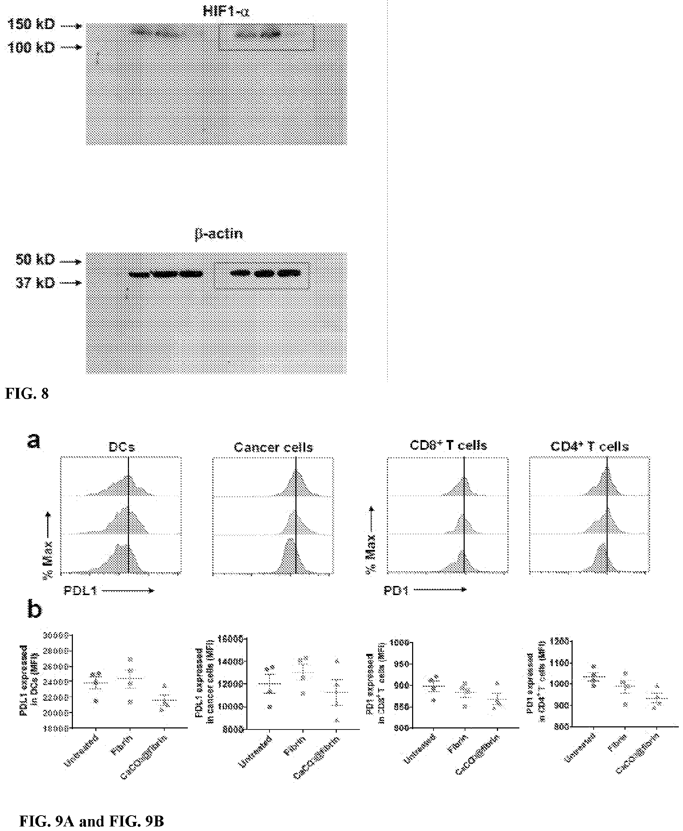

17. Figure 8 shows uncropped western blots for Figure 7G. Lanes used for Figure 7G are indicated by red rectangles.

18. Figures 9A and 9B show the PD-L1 expression of DCs and tumor cells and PD-1 expression of tumor-infiltrating lymphocytes (9A) after CaCO3@Fibrin treatment and (9B) the corresponding quantification of PD-L1 and PD-1mean intensity. The error bars represent standard error of the mean (s.e.m.) (n = 4). Statistical significance was calculated via one-way ANOVA with a Tukey post-hoc test. P value: *P < 0.05; **P < 0.01; ***P < 0.005.

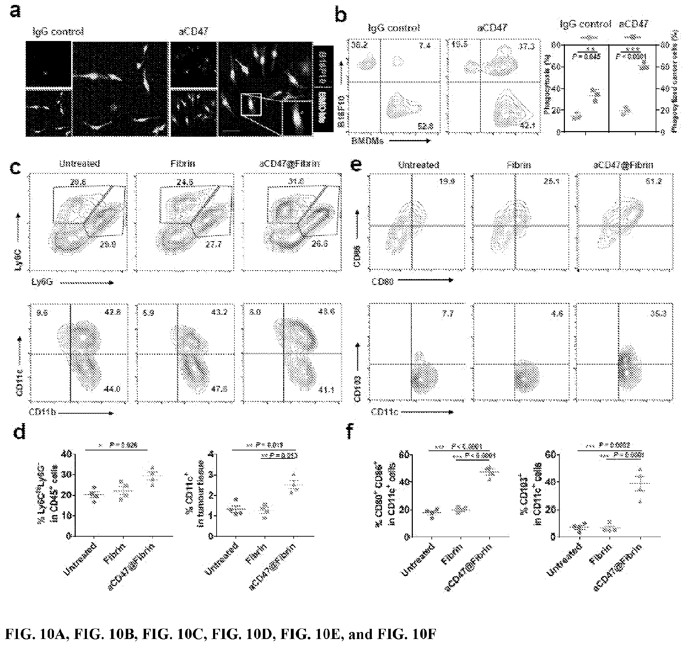

19. Figure 10A, 10B, 10C, 10D, 10E, and 10F show CD47 blockade for increasing phagocytosis in vitro and exerting antitumor immune responses in vivo. Figure 10A shows representative confocal images of phagocytosis assays, in which B 16F 10 cancer cells were labeled with CellTracker DeepRed (red) and BMDMs were labeled with CellTracker Green (green) (Scale bar, 100 µm). Figure 10B shows representative flow cytometric analysis images (left) and corresponding quantification (right) of the phagocytosis of cancer cells by BMDMs (n = 3). Phagocytized cancer cells were quantified as the percentage of double-positive BMDMs among CellTracker Green-positive BMDMs or CellTracker DeepRed-cancer cells. Figure 10C shows representative flow cytometric analysis images and (10D) corresponding quantification of Ly6ChiLy6G- macrophages and CD11c+CD11b- DCs gating on CD45+ cells. Figure 10E shows representative flow cytometric analysis images and 10F) corresponding quantification of CD80+CD86+ DCs and CD103+ DCs gating on CD11c+ cells. The error bars represent standard error of the mean (s.e.m.) (n = 4). Statistical significance was calculated via one-way ANOVA with a Tukey post-hoc test. P value: *P < 0.05; **P < 0.01; ***P < 0.001.

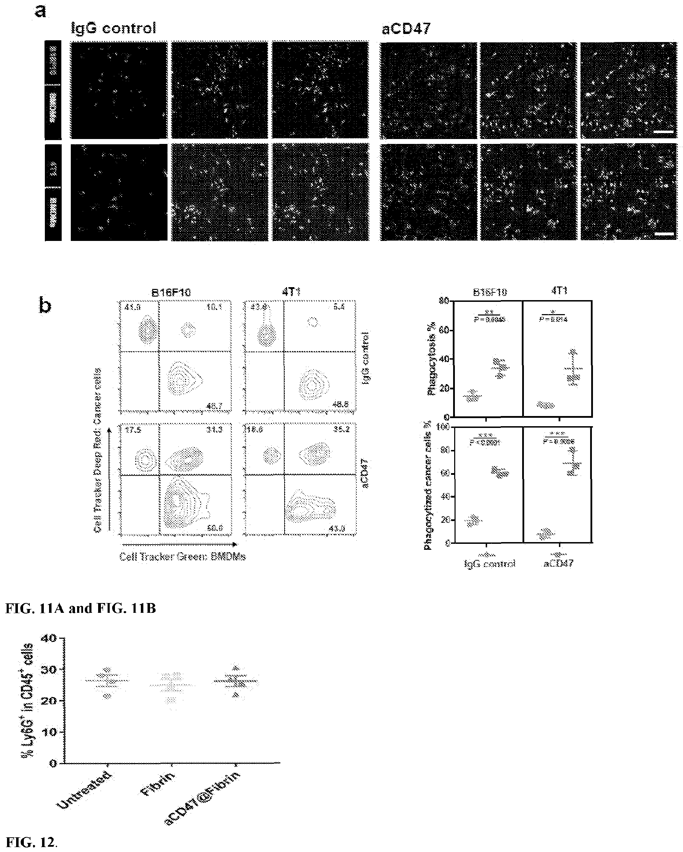

20. Figure 11A shows representative confocal images of phagocytosis assays in which cancer cells including B16F10 and 4T1 were labeled with CellTracker DeepRed and BMDMs were labeled with CellTracker Green (Scale bar, 100 µm).

21. Figure 11B shows representative flow cytometric analysis images (left) and corresponding quantification (right) of the phagocytosis of cancer cells (B16F10 and 4T1) by BMDMs (n = 3). Phagocytized cancer cells was quantified as the percentage of double-positive BMDMs among CellTracker Green-positive BMDMs or CellTracker DeepRed-cancer cells. The error bars represent standard error of the mean (s.e.m.) (n = 3). Statistical significance was calculated via one-way ANOVA with a Tukey post-hoc test. P value: *P < 0.05; **P < 0.01; ***P < 0.005.

22. Figure 12 shows the percentage of Ly6G+Ly6Cdim neutrophils gating on CD45+ cells.

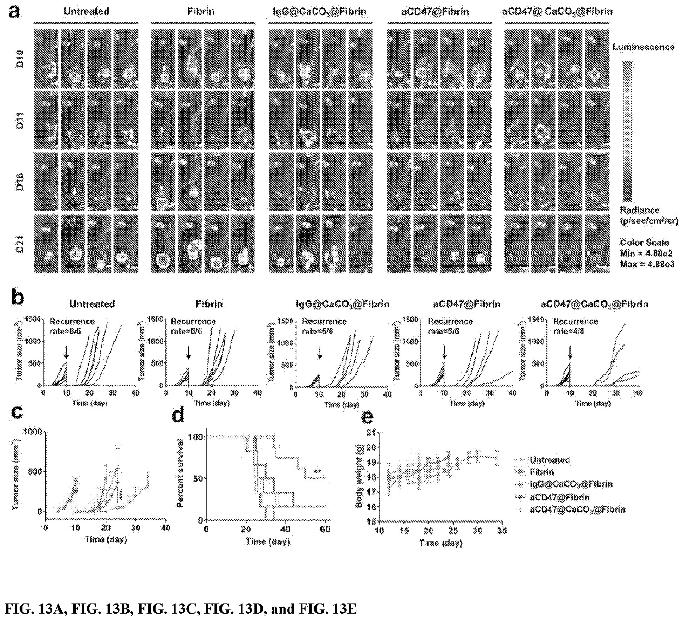

23. Figures 13A, 13B, and 13C shows aCD47@CaCO3@Fibrin for reducing recurrence of B16F10 tumors after surgery. Figure 13A shows in vivo bioluminescence imaging of B16F10 tumors after removal of the primary tumor. Four representative mice per treatment group are shown. Figure 13B shows individual and (13C) average tumor growth kinetics in different groups. Growth curves were stopped when the first mouse of the corresponding group died. Statistical significance was calculated via two-way ANOVA with a Tukey post-hoc test. Figure 13D shows the survival corresponding to the tumor size of mice after different treatments as indicated. tatistical significance was calculated via Log-Rank (Mantel-Cox) test. Figure 13E shows weight changes of the mice in different groups. The error bars represent standard error of the mean (s.e.m.) (n = 6-8). Statistical significance was calculated via two-way ANOVA with a Tukey post-hoc test. P value: *P < 0.05; **P < 0.01; ***P < 0.001.

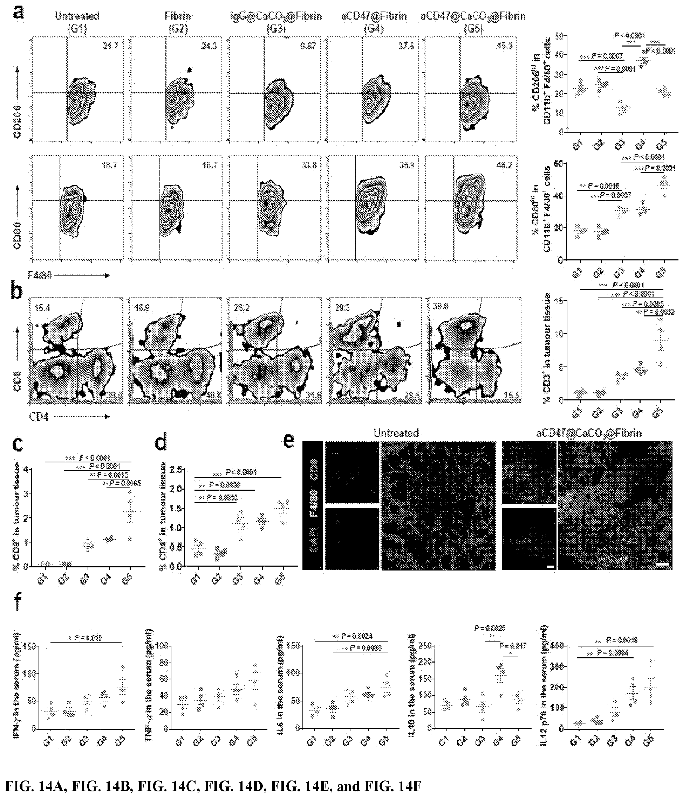

24. Figures 14A, 14B, 14C, 14D, 14E, and 14F shows aCD47@CaCO3@Fibrin triggering a robust antitumor immune response. B16F10 tumors were harvested from mice five days after different treatments for analysis. Figure 14A shows representative flow cytometric analysis images (left) and corresponding quantification (right) of M2-macrophages (CD206hi) and M1-macrophages (CD80 hi) in F4/80+CD11b+ CD45+ cells. Figure 14B shows representative flow cytometric analysis of T cell infiltration within the tumor (left) and corresponding quantification results (right) in different groups. Figure 14C shows absolute percentage of the CD8+ and (14D) CD4+ T cells within tumors upon various treatments. Figure 14E shows representative immunofluorescence images of tumors showing CD8+ T cell and F4/80+ macrophage infiltration. Scale bar, 50 µm. Figure 14F shows cytokine levels in the sera from mice isolated at five days after different treatments. Error bars represent the s.e.m. (n = 4). G1: Untreated, G2: Fibrin, G3: IgG@CaCO3@Fibrin, G4: aCD47@Fibrin, G5: aCD47@CaCO3@Fibrin. Statistical significance was calculated via two-way ANOVA with a Tukey post-hoc test. P value: *P < 0.05; **P < 0.01; ***P < 0.001.

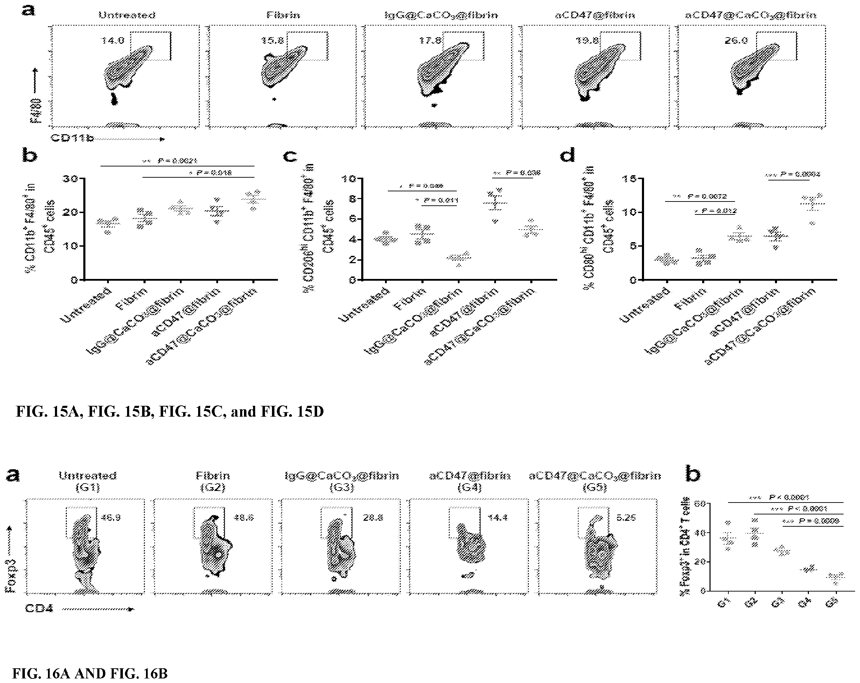

25. Figure 15A and 15B show representative flow cytometric analysis images (15A) and the corresponding quantification (15B) of F4/80+CD11b+ macrophages in CD45+ cells.

26. Figure 15C and 15D show the percentage of (15C) M2-macrophages (CD206hi) and (15D) M1-macrophages (CD80 hi) in CD45+ cells. The error bars represent standard error of the mean (s.e.m.) (n = 4). Statistical significance was calculated via two-way ANOVA with a Tukey post-hoc test. P value: *P < 0.05; **P < 0.01; ***P < 0.005.

27. Figure 16A shows representative flow cytometric analysis images of CD4+Foxp3+ T cells gating on CD3+ cells after different treatments.

28. Figure 16B shows the corresponding quantification of CD4+Foxp3+ T cells gating on CD3+ cells after different treatments.

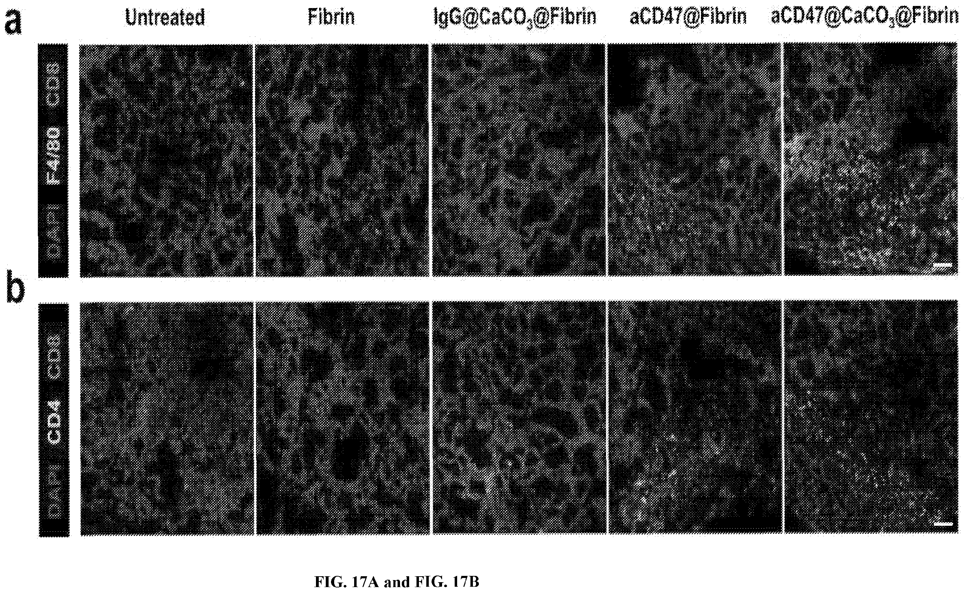

29. Figures 17A and 17B shows representative immunofluorescence images of tumors showing CD4+ T cell, CD8+ T cell, and F4/80+ macrophages infiltration. Scale bar, 50 µm.

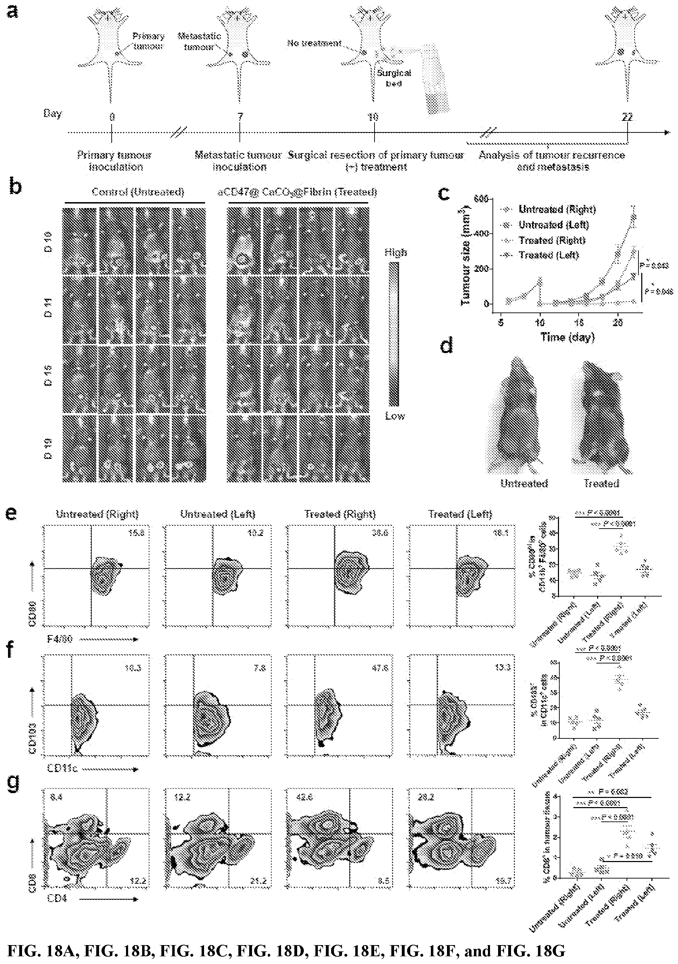

30. Figures 18A, 18B, 18C, 18D, 18E, 18F, and 18G show the local treatment of aCD47@CaCO3@Fibrin inducing systemic antitumor immune response. Figure 18A shows a schematic illustrating aCD47@CaCO3@Fibrin therapy in a mouse model of incomplete resection and metastasis. Tumors on the right side were designated as "primary tumors" for aCD47@CaCO3@Fibrin treatment, and those on the left side were designated as "distant tumors" without any treatment. Figure 18B shows in vivo bioluminescence imaging of B16F10 tumors in response to local aCD47@CaCO3@Fibrin treatment. Figure 18C shows growth curves for left and right tumors in untreated and treated mice (n = 8). Figure 18D shows representative mice photographs on day 22. Red arrows indicate the tumors. Figure 18E shows representative flow cytometric analysis images (left) and corresponding quantification (right) of M1-macrophages (CD80hi) in F4/80+CD11b+CD45+ cells (n = 4). Figure 18F shows representative flow cytometric analysis images (left) and corresponding quantification (right) of CD103+ DCs in CD11c+ cells (n = 4). Figure 18G shows representative flow cytometric analysis of T cell infiltration within the tumor (left) and percentage (right) of the CD8+ T cells in different groups (n = 4). The error bars represent standard error of the mean (s.e.m.). Statistical significance was calculated via one-way ANOVA with a Tukey post-hoc test. P value: *P < 0.05; **P < 0.01; ***P < 0.001.

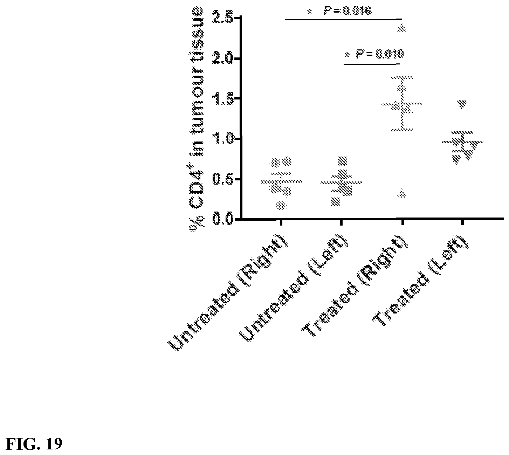

31. Figure 19 shows the percentage of the CD4+ cells in left and right tumors in untreated and treated mice. The error bars indicate standard error of the mean (s.e.m.). Statistical significance was calculated by one-way ANOVA using the Tukey post-test. P value: *, P<0.05; **, P<0.01; ***P<0.005.

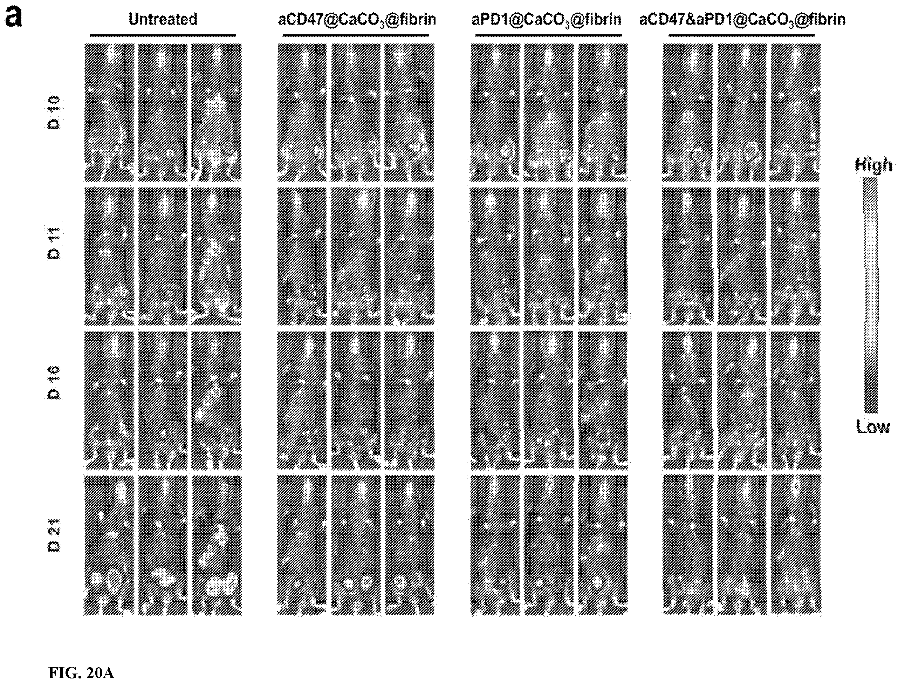

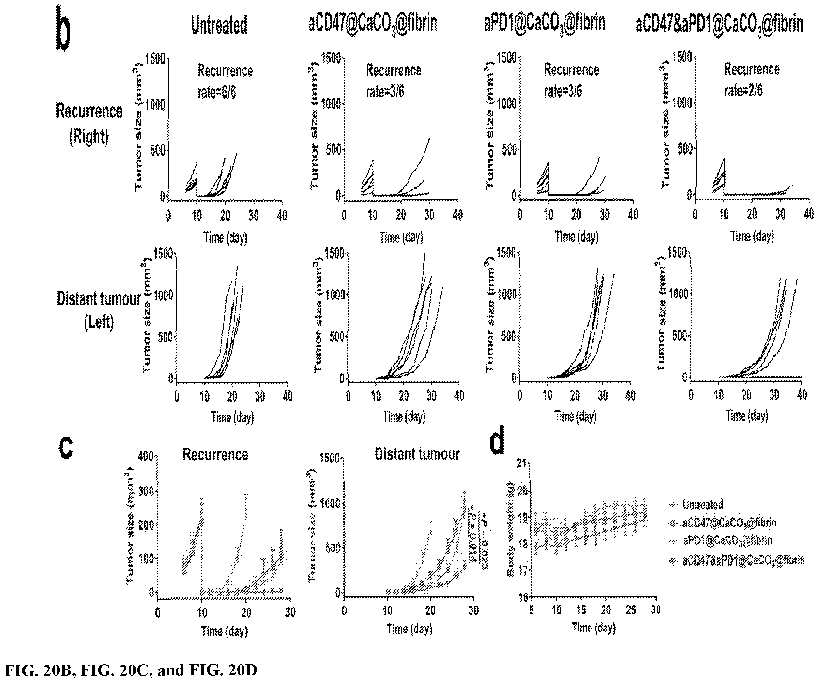

32. Figures 20A, 20B, 20C, and 20D show inhibition of tumor recurrence and distant tumors with combination immunotherapy. Figure 20A shows *In vivo* bioluminescence imaging of B16F 10 tumors after different treatments. The experiments were repeated three times. (Figure 20B shows individual and Figure 20C shows average tumor growth kinetics in different groups. Data are presented as mean ± s.e.m. (*n*=6). Growth curves were interrupted when the first mouse of the corresponding group was euthanized. Figure 20C shows weight changes of the mice in different groups. Data are presented as mean ± s.e.m. (*n*=6). Statistical significance was calculated *via* one-way ANOVA with a Tukey post-hoc test. *P* value: **P* < 0.05.

### IV. DETAILED DESCRIPTION

33. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### A. Definitions

34. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

35. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

36. Administration" to a subject includes any route of introducing or delivering to a subject an agent. Administration can be carried out by any suitable route, including oral, topical, intravenous, subcutaneous, transcutaneous, transdermal, intramuscular, intra-joint, parenteral, intra-arteriole, intradermal, intraventricular, intracranial, intraperitoneal, intralesional, intranasal, rectal, vaginal, by inhalation, via an implanted reservoir, parenteral (e.g., subcutaneous, intravenous, intramuscular, intra-articular, intra-synovial, intrasternal, intrathecal, intraperitoneal, intrahepatic, intralesional, and intracranial injections or infusion techniques), and the like. "Concurrent administration", "administration in combination", "simultaneous administration" or "administered simultaneously" as used herein, means that the compounds are administered at the same point in time or essentially immediately following one another. In the latter case, the two compounds are administered at times sufficiently close that the results observed are indistinguishable from those achieved when the compounds are administered at the same point in time. "Systemic administration" refers to the introducing or delivering to a subject an agent via a route which introduces or delivers the agent to extensive areas of the subject's body (e.g. greater than 50% of the body), for example through entrance into the circulatory or lymph systems. By contrast, "local administration" refers to the introducing or delivery to a subject an agent via a route which introduces or delivers the agent to the area or area immediately adjacent to the point of administration and does not introduce the agent systemically in a therapeutically significant amount. For example, locally administered agents are easily detectable in the local vicinity of the point of administration, but are undetectable or detectable at negligible amounts in distal parts of the subject's body. Administration includes self-administration and the administration by another.

37. "Biocompatible" generally refers to a material and any metabolites or degradation products thereof that are generally non-toxic to the recipient and do not cause significant adverse effects to the subject.

38. "Comprising" is intended to mean that the compositions, methods, etc. include the recited elements, but do not exclude others. "Consisting essentially of" when used to define compositions and methods, shall mean including the recited elements, but excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions of this invention. Embodiments defined by each of these transition terms are within the scope of this disclosure.

39. A "control" is an alternative subject or sample used in an experiment for comparison purposes. A control can be "positive" or "negative."

40. "Controlled release" or "sustained release" refers to release of an agent from a given dosage form in a controlled fashion in order to achieve the desired pharmacokinetic profile in vivo. An aspect of "controlled release" agent delivery is the ability to manipulate the formulation and/or dosage form in order to establish the desired kinetics of agent release.

41. "Effective amount" of an agent refers to a sufficient amount of an agent to provide a desired effect. The amount of agent that is "effective" will vary from subject to subject, depending on many factors such as the age and general condition of the subject, the particular agent or agents, and the like. Thus, it is not always possible to specify a quantified "effective amount." However, an appropriate "effective amount" in any subject case may be determined by one of ordinary skill in the art using routine experimentation. Also, as used herein, and unless specifically stated otherwise, an "effective amount" of an agent can also refer to an amount covering both therapeutically effective amounts and prophylactically effective amounts. An "effective amount" of an agent necessary to achieve a therapeutic effect may vary according to factors such as the age, sex, and weight of the subject. Dosage regimens can be adjusted to provide the optimum therapeutic response. For example, several divided doses may be administered daily or the dose may be proportionally reduced as indicated by the exigencies of the therapeutic situation.

42. "Pharmaceutically acceptable" component can refer to a component that is not biologically or otherwise undesirable, i.e., the component may be incorporated into a pharmaceutical formulation of the invention and administered to a subject as described herein without causing significant undesirable biological effects or interacting in a deleterious manner with any of the other components of the formulation in which it is contained. When used in reference to administration to a human, the term generally implies the component has met the required standards of toxicological and manufacturing testing or that it is included on the Inactive Ingredient Guide prepared by the U.S. Food and Drug Administration.

43. "Pharmaceutically acceptable carrier" (sometimes referred to as a "carrier") means a carrier or excipient that is useful in preparing a pharmaceutical or therapeutic composition that is generally safe and non-toxic, and includes a carrier that is acceptable for veterinary and/or human pharmaceutical or therapeutic use. The terms "carrier" or "pharmaceutically acceptable carrier" can include, but are not limited to, phosphate buffered saline solution, water, emulsions (such as an oil/water or water/oil emulsion) and/or various types of wetting agents. As used herein, the term "carrier" encompasses, but is not limited to, any excipient, diluent, filler, salt, buffer, stabilizer, solubilizer, lipid, stabilizer, or other material well known in the art for use in pharmaceutical formulations and as described further herein.

44. "Pharmacologically active" (or simply "active"), as in a "pharmacologically active" derivative or analog, can refer to a derivative or analog (e.g., a salt, ester, amide, conjugate, metabolite, isomer, fragment, etc.) having the same type of pharmacological activity as the parent compound and approximately equivalent in degree.

45. "Therapeutic agent" refers to any composition that has a beneficial biological effect. Beneficial biological effects include both therapeutic effects, e.g., treatment of a disorder or other undesirable physiological condition, and prophylactic effects, e.g., prevention of a disorder or other undesirable physiological condition (e.g., a non-immunogenic cancer). The terms also encompass pharmaceutically acceptable, pharmacologically active derivatives of beneficial agents specifically mentioned herein, including, but not limited to, salts, esters, amides, proagents, active metabolites, isomers, fragments, analogs, and the like. When the terms "therapeutic agent" is used, then, or when a particular agent is specifically identified, it is to be understood that the term includes the agent per se as well as pharmaceutically acceptable, pharmacologically active salts, esters, amides, proagents, conjugates, active metabolites, isomers, fragments, analogs, etc.

46. "Polymer" refers to a relatively high molecular weight organic compound, natural or synthetic, whose structure can be represented by a repeated small unit, the monomer. Non-limiting examples of polymers include polyethylene, rubber, cellulose. Synthetic polymers are typically formed by addition or condensation polymerization of monomers. The term "copolymer" refers to a polymer formed from two or more different repeating units (monomer residues). By way of example and without limitation, a copolymer can be an alternating copolymer, a random copolymer, a block copolymer, or a graft copolymer. It is also contemplated that, in certain aspects, various block segments of a block copolymer can themselves comprise copolymers. The term "polymer" encompasses all forms of polymers including, but not limited to, natural polymers, synthetic polymers, homopolymers, heteropolymers or copolymers, addition polymers, etc.

47. "Therapeutically effective amount" or "therapeutically effective dose" of a composition (e.g. a composition comprising an agent) refers to an amount that is effective to achieve a desired therapeutic result. In some embodiments, a desired therapeutic result is the control of type I diabetes. In some embodiments, a desired therapeutic result is the control of obesity. Therapeutically effective amounts of a given therapeutic agent will typically vary with respect to factors such as the type and severity of the disorder or disease being treated and the age, gender, and weight of the subject. The term can also refer to an amount of a therapeutic agent, or a rate of delivery of a therapeutic agent (e.g., amount over time), effective to facilitate a desired therapeutic effect, such as pain relief. The precise desired therapeutic effect will vary according to the condition to be treated, the tolerance of the subject, the agent and/or agent formulation to be administered (e.g., the potency of the therapeutic agent, the concentration of agent in the formulation, and the like), and a variety of other factors that are appreciated by those of ordinary skill in the art. In some instances, a desired biological or medical response is achieved following administration of multiple dosages of the composition to the subject over a period of days, weeks, or years.

48. In this specification and in the claims which follow, reference will be made to a number of terms which shall be defined to have the following meanings:

49. "Optional" or "optionally" means that the subsequently described event or circumstance may or may not occur, and that the description includes instances where said event or circumstance occurs and instances where it does not.

50. Throughout this application, various publications are referenced.

### B. Compositions

51. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular hydrogel matrix comprising a chemotherapeutic agent and a blockade inhibitor is disclosed and discussed and a number of modifications that can be made to a number of molecules including the hydrogel matrix comprising a chemotherapeutic agent and a blockade inhibitor are discussed, specifically contemplated is each and every combination and permutation of hydrogel matrix comprising a chemotherapeutic agent and a blockade inhibitor and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

52. Recent successes of cancer immunotherapy suggest that it can be utilized to prevent cancer recurrence. In one aspect, disclosed herein are immunotherapeutic bioresponsive gel matrixes comprising a first component (for example, a H⁺ scavenger such as, for example, fibrinin) and a second component (such as, for example, thrombin); wherein gelation of the matrix occurs when the first component comes into contact with the second component; and wherein at least one of the components (i.e., the first component and/or second component) comprises at least one first nanoparticle; wherein the at least one first nanoparticle comprises at least one therapeutic agent cargo such, as example, an anti-cancer agent, blockade inhibitor, or immunomodulatory agent. In one aspect, the at least one first nanoparticle can comprise a pH responsive material such as, for example, dextran, CaCO3, chitosan, hyaluronic acid, as well as polymers thereof including, for example polymers of dextran monomers (for example a polymer of m-dextran monomers).

53. It is understood and herein contemplated that the therapeutic agent cargo can comprise an antibody, small molecule, peptide, polypeptide, peptide mimetic, polymer, or nucleic acid. For example, the therapeutic agent cargo can comprise an immune checkpoint inhibitor such as for example, a PD-1 inhibitor, a PD-L1 inhibitor, or CTLA-4 inhibitor (such as, for example, nivolumab, pembrolizumab, pidilizumab, BMS-936559, Atezolizumab, Durvalumab, or Avelumab); an immunomodulatory agent such as, for example, any antibody, cytokine, chemokine, nucleic acid, or small molecule the inhibits or promotes the activity, production, proliferation, or maturation of immune cells including but not limited to NK cells, NK T cells, T cells, B cells, plasma cells, macrophage, dendritic cells, and mast cells. In one aspect, the immunomudulator agent can be IFN-γ, TNF-α, TGF-β, IL-1β, IL-2 IL-4, IL-5, IL-6, IL-10, IL-12, anti-CD3 antibody, anti-CD4 antibody, anti-CD8 anti-body, anti-CTLA-4 antibody, anti-4-1BB antibody, anti-OX40 antibody, anti-CD25 antibody, anti-CD27 antibody, anti-LAG3 antibody, and/or anti-CD47 antibody; and/or one or more chemotherapeutic agents. Chemotherapeutic agents that can be used in the disclosed hydrogel matrixes can comprise any anti-cancer agent known in the art, the including, but not limited to Abemaciclib, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin Chlorambucil), Amifostine, Aminolevulinic Acid, Anastrozole, Aprepitant, Aredia (Pamidronate Disodium), Arimidex (Anastrozole), Aromasin (Exemestane),Arranon (Nelarabine), Arsenic Trioxide, Arzerra (Ofatumumab), Asparaginase Erwinia chrysanthemi, Atezolizumab, Avastin (Bevacizumab), Avelumab, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin) , Bevacizumab, Bexarotene, Bexxar (Tositumomab and Iodine I 131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Campath (Alemtuzumab), Camptosar, (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride , EPOCH, Erbitux (Cetuximab), Eribulin Mesylate, Erivedge (Vismodegib), Erlotinib Hydrochloride, Erwinaze (Asparaginase Erwinia chrysanthemi) , Ethyol (Amifostine), Etopophos (Etoposide Phosphate), Etoposide, Etoposide Phosphate, Evacet (Doxorubicin Hydrochloride Liposome), Everolimus, Evista , (Raloxifene Hydrochloride), Evomela (Melphalan Hydrochloride), Exemestane, 5-FU (Fluorouracil Injection), 5-FU (Fluorouracil-Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil--Topical), Fluorouracil Injection, Fluorouracil--Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride) , Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride, Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and , Hyaluronidase Human, ,Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq , (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil--Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Vandetanib, VAMP, Varubi (Rolapitant Hydrochloride), Vectibix (Panitumumab), VeIP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), and/or Zytiga (Abiraterone Acetate).

54. Macrophages, an important component of innate immunity, are able to phagocytize foreign substances without specific "self-signal" protein on their surfaces and present them to T lymphocytes (T cells). However, tricky cancer cells up-regulate the integrin-associated protein (IAP), also called CD47, an antiphagocytic "don't eat me" signal, to avoid phagocytosis by macrophages. Blocking the interaction between CD47 and its ligand, signal regulatory protein-*α* (SIRP*α*) expressed on macrophages, dendritic cells (DCs) and neutrophils, activate these phagocytic cells and promote the phagocytosis of cancer cells. Despite significant investment and remarkable progress in phase I clinical trials, the current CD47 blockade strategy still has many limitations. Antibody-dependent cell mediated cytotoxicity (ADCC) usually limits their applications. Efforts to avoid these severe side effects are highly needed in the field of CD47-mediated immunotherapies. In one aspect, disclosed herein are immunotherapeutic bioresponsive gel matrixes comprising a first component (for example, a H+ scavenger such as, for example, fibrinin) and a second component (such as, for example thrombin); wherein gelation of the matrix occurs when the first component comes into contact with the second component; and wherein at least one the components (i.e., the first component and/or second component) comprises at least one first nanoparticle; wherein the at least one first nanoparticle comprises at least one anti-cancer agent, blockade inhibitor, or immunomodulatory agent; wherein the at least one anti-cancer agent, blockade inhibitor, or immunomodulatory agent comprises an anti-CD47 antibody.

55. As noted above, the disclosed immunotherapeutic bioresponsive gel matrixes comprises a nanoparticle designed for extended release of at least one anti-cancer agent, blockade inhibitor, or immunomodulatory agent. It is understood and herein contemplated that the choice of which component of the immunotherapeutic bioresponsive gel matrixe (i.e., the first or second component) comprises the nanoparticle can be a decision having more to do with manufacture and storage concerns rather than any medical effect. Accordingly, it is understood that either the first component, second component, or both components can comprise a nanoparticle.

56. In one aspect, disclosed herein the immunotherapeutic bioresponsive gel matrixes can comprise more than one nanoparticle. For example, the immunotherapeutic bioresponsive gel matrix can comprise at least 2, 3, 4, 5, 6, 7, 8, 9, or 10 nanoparticles. Thus, in one aspect, the bioresponsive gel matrix can comprise at least one first, second, third, fourth, fifth, sixth, seventh, eighth, ninth, and/or tenth nanoparticle. It is further understood and herein contemplated that the first and any subsequent nanoparticle can be comprised in the first component, second component, or both components. In one aspect, the first nanoparticle and any subsequent nanoparticle (for example, the second nanoparticle) are comprised in the same component. In another aspect, the first nanoparticle and any subsequent nanoparticle (for example, the second nanoparticle) are comprised in different components. For example, disclosed herein are bioresponsive gel matrixes wherein the first component comprises the first nanoparticle and the second component comprises at least one second nanoparticle. Also disclosed herein are bioresponsive gel matrixes wherein the first component comprises at least one first nanoparticle and at least one second nanoparticle. In one aspect, disclosed herein are bioresponsive gel matrixes wherein the second component comprises at least one first nanoparticle and at least one second nanoparticle. Also disclosed are bioresponsive gel matrixes wherein the second component comprises the first nanoparticle and the first component comprises at least one subsequent nanoparticle (for example a second nanoparticle).

57. It is further understood and herein contemplated that the components of the bioresponsive gel matrixes can comprise more than one type of anti-cancer agent, blockade inhibitor, or immunomodulatory agent. In one aspect, the nanoparticles can comprise more than one type of anti-cancer agent, blockade inhibitor, or immunomodulatory agent and that said anti-cancer agent, blockade inhibitor, or immunomodulatory agent. For example, the nanoparticle can comprise any combination of 1, 2, 3, 4, 5, 6, 7, 8, 9,10, 11, 12, 13, 14, 15, 16, 17, 18, 19, or 20 anti-cancer agents, blockade inhibitors, or immunomodulatory agents. It is understood and herein contemplated that as the bioresponsive gel matrix can comprises one or more nanoparticles, each nanoparticle can comprise any combination of 1, 2, 3, 4, 5, 6, 7, 8, 9,10, 11, 12, 13, 14, 15, 16, 17, 18, 19, or 20 anti-cancer agents, blockade inhibitors, or immunomodulatory agents. Additionally, it is understood and herein contemplated that in addition to the one or more anti-cancer agents, blockade inhibitors, or immunomodulatory agents comprised within any of the nanoparticles of first and/or second component, the immunotherapeutic bioresponsive gel matrix can also comprise an additional 1, 2, 3, 4, 5, 6, 7, 8, 9, 10, 11, 12, 13, 14, 15, 16, 17, 18, 19, or 20 anti-cancer agents, blockade inhibitors, or immunomodulatory agents. It is also understood and herein contemplated that any additional anti-cancer agents, blockade inhibitors, or immunomodulatory agents comprised within a nanoparticle can be comprised in the same or different component that any first anti-cancer agents, blockade inhibitors, or immunomodulatory agents either provided in the first nanoparticle or external to any first nanoparticle.

58. To facilitate these functions, the bioresponsive hydrogel can be engineered as a polymer. "Polymer" refers to a relatively high molecular weight organic compound, natural or synthetic, whose structure can be represented by a repeated small unit, the monomer. Non-limiting examples of polymers include polyethylene, rubber, cellulose. Synthetic polymers are typically formed by addition or condensation polymerization of monomers. The term "copolymer" refers to a polymer formed from two or more different repeating units (monomer residues). By way of example and without limitation, a copolymer can be an alternating copolymer, a random copolymer, a block copolymer, or a graft copolymer. It is also contemplated that, in certain aspects, various block segments of a block copolymer can themselves comprise copolymers. The term "polymer" encompasses all forms of polymers including, but not limited to, natural polymers, synthetic polymers, homopolymers, heteropolymers or copolymers, addition polymers, etc. In one aspect, the gel matrix can comprise copolymers, block copolymers, diblock copolymers, and/or triblock copolymers.

59. The triblock copolymers disclosed herein comprise a core polymer such as, example, polyethylene glycol (PEG), polyvinyl acetate, polyvinyl alcohol, polyvinyl pyrrolidone (PVP), polyethyleneoxide (PEO), poly(vinyl pyrrolidone-co-vinyl acetate), polymethacrylates, polyoxyethylene alkyl ethers, polyoxyethylene castor oils, polycaprolactam, polylactic acid, polyglycolic acid, poly(lactic-glycolic) acid, poly(lactic co-glycolic) acid (PLGA), cellulose derivatives, such as hydroxymethylcellulose, hydroxypropylcellulose and the like. In one aspect, the core polymer can be flanked by polypeptide blocks.

60. The immunotherapeutic bioresponsive gel matrixes disclosed herein not only serve as a localized drug delivery depot for efficiently transporting therapeutics, but also modulates the intratumoral microenvironment for promoting effectiveness of therapy. For example, the H+ scavenger (such as, for example, Fibrinin) in the first component can raise the pH of the microenvironment, alter reactive oxygen species, or modulate T cells to a TH1 or TH2 type or macrophage to a M1 type. For example, the modulation of the pH in the tumor microenvironment (TME), can effect the release rate of any anti-cancer agent, blockade inhibitor, and/or immunotherapeutic agent comprised in a nanoparticle or otherwise contained within the immunotherapeutic bioresponsive gel matrix. As shown herein, 50% of the anti-cancer agent, blockade inhibitor, and/or immunotherapeutic agent was released by 24 hours and approximately 80% was released by 140 hours where the pH of the tumor microenvironment is is 6.5. However, by raising the pH of the TME to a pH 7.4 and thus, more closely reflecting the pH of blood, the release of the anti-cancer agent, blockade inhibitor, and/or immunotherapeutic agent was shown to be significantly extended to approximately 20% release at 140 hours. In one aspect, disclosed herein are any of the bioresponsive gel matrixes disclosed herein wherein less than 80, 75, 70, 65, 60, 55, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, or 10% of the anti-cancer agent, blockade inhibitor, and/or immunotherapeutic agent has been released at 140 hours. In one aspect, the between 10-50% of the anti-cancer agent, blockade inhibitor, and/or immunotherapeutic agent has been released at 140 hours, preferably between 20-40% of the anti-cancer agent, blockade inhibitor, and/or immunotherapeutic agent has been released at 140 hours. It is understood and herein contemplated that release rate can also be referenced herein by its converse, i.e., the retention rate (the amount retained at a time point). Accordingly, in one aspect, disclosed herein are any of the bioresponsive gel matrixes disclosed herein wherein at least 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or 90% of the anti-cancer agent, blockade inhibitor, and/or immunotherapeutic agent is retained at 140 hours.

### 1. Antibodies

### (1) Antibodies Generally

61. The term "antibodies" is used herein in a broad sense and includes both polyclonal and monoclonal antibodies. In addition to intact immunoglobulin molecules, also included in the term "antibodies" are fragments or polymers of those immunoglobulin molecules, and human or humanized versions of immunoglobulin molecules or fragments thereof. The antibodies can be tested for their desired activity using the *in vitro* assays described herein, or by analogous methods, after which their *in vivo* therapeutic and/or prophylactic activities are tested according to known clinical testing methods. There are five major classes of human immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. One skilled in the art would recognize the comparable classes for mouse. The heavy chain constant domains that correspond to the different classes of immunoglobulins are called alpha, delta, epsilon, gamma, and mu, respectively.

62. The term "monoclonal antibody" as used herein refers to an antibody obtained from a substantially homogeneous population of antibodies, i.e., the individual antibodies within the population are identical except for possible naturally occurring mutations that may be present in a small subset of the antibody molecules. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity.

63. The disclosed monoclonal antibodies can be made using any procedure which produces mono clonal antibodies. For example, disclosed monoclonal antibodies can be prepared using hybridoma methods, such as those described by Kohler and Milstein, Nature, 256:495 (1975). In a hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro.*

64. The monoclonal antibodies may also be made by recombinant DNA methods. DNA encoding the disclosed monoclonal antibodies can be readily isolated and sequenced using conventional procedures (e.g., by using oligonucleotide probes that are capable of binding specifically to genes encoding the heavy and light chains of murine antibodies). Libraries of antibodies or active antibody fragments can also be generated and screened using phage display techniques, e.g., as described in U.S. Patent No. 5,804,440 to Burton et al. and U.S. Patent No. 6,096,441 to Barbas et al.

65. *In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published Dec. 22, 1994 and U.S. Pat. No. 4,342,566. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

66. As used herein, the term "antibody or fragments thereof' encompasses chimeric antibodies and hybrid antibodies, with dual or multiple antigen or epitope specificities, and fragments, such as F(ab')2, Fab', Fab, Fv, scFv, and the like, including hybrid fragments. Thus, fragments of the antibodies that retain the ability to bind their specific antigens are provided. Such antibodies and fragments can be made by techniques known in the art and can be screened for specificity and activity according to the methods set forth in the Examples and in general methods for producing antibodies and screening antibodies for specificity and activity (See Harlow and Lane. Antibodies, A Laboratory Manual. Cold Spring Harbor Publications, New York, (1988)).

67. Also included within the meaning of "antibody or fragments thereof' are conjugates of antibody fragments and antigen binding proteins (single chain antibodies).

68. The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment. (Zoller, M.J. Curr. Opin. Biotechnol. 3:348-354, 1992).

69. As used herein, the term "antibody" or "antibodies" can also refer to a human antibody and/or a humanized antibody. Many non-human antibodies (e.g., those derived from mice, rats, or rabbits) are naturally antigenic in humans, and thus can give rise to undesirable immune responses when administered to humans. Therefore, the use of human or humanized antibodies in the methods serves to lessen the chance that an antibody administered to a human will evoke an undesirable immune response.

### (2) Human antibodies

70. The disclosed human antibodies can be prepared using any technique. The disclosed human antibodies can also be obtained from transgenic animals. For example, transgenic, mutant mice that are capable of producing a full repertoire of human antibodies, in response to immunization, have been described (see, e.g., Jakobovits et al., Proc. Natl. Acad. Sci. USA, 90:2551-255 (1993); Jakobovits et al., Nature, 362:255-258 (1993); Bruggermann et al., Year in Immunol., 7:33 (1993)). Specifically, the homozygous deletion of the antibody heavy chain joining region (J*(H)*) gene in these chimeric and germ-line mutant mice results in complete inhibition of endogenous antibody production, and the successful transfer of the human germ-line antibody gene array into such germ-line mutant mice results in the production of human antibodies upon antigen challenge. Antibodies having the desired activity are selected using Env-CD4-co-receptor complexes as described herein.

### (3) Humanized antibodies

71. Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an sFv, Fv, Fab, Fab', F(ab')2, or other antigen-binding portion of an antibody) which contains a portion of an antigen binding site from a non-human (donor) antibody integrated into the framework of a human (recipient) antibody.

72. To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones et al., Nature, 321:522-525 (1986), Reichmann et al., Nature, 332:323-327 (1988), and Presta, Curr. Opin. Struct. Biol., 2:593-596 (1992)).

73. Methods for humanizing non-human antibodies are well known in the art. For example, humanized antibodies can be generated according to the methods of Winter and co-workers (Jones et al., Nature, 321:522-525 (1986), Riechmann et al., Nature, 332:323-327 (1988), Verhoeyen et al., Science, 239:1534-1536 (1988)), by substituting rodent CDRs or CDR sequences for the corresponding sequences of a human antibody. Methods that can be used to produce humanized antibodies are also described in U.S. Patent No. 4,816,567 (Cabilly et al.), U.S. Patent No. 5,565,332 (Hoogenboom et al.), U.S. Patent No. 5,721,367 (Kay et al.), U.S. Patent No. 5,837,243 (Deo et al.), U.S. Patent No. 5, 939,598 (Kucherlapati et al.), U.S. Patent No. 6,130,364 (Jakobovits et al.), and U.S. Patent No. 6,180,377 (Morgan et al.).

### (4) Administration of antibodies

74. Administration of the antibodies can be done as disclosed herein. Nucleic acid approaches for antibody delivery also exist. The broadly neutralizing antibodies and antibody fragments can also be administered to patients or subjects as a nucleic acid preparation (e.g., DNA or RNA) that encodes the antibody or antibody fragment, such that the patient's or subject's own cells take up the nucleic acid and produce and secrete the encoded antibody or antibody fragment. The delivery of the nucleic acid can be by any means, as disclosed herein, for example.

### 2. Pharmaceutical carriers/Delivery of pharamceutical products

75. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

76. The compositions may be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

77. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795.

78. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

79. The compositions, including antibodies, can be used therapeutically in combination with a pharmaceutically acceptable carrier.

80. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

81. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

82. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

83. The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

84. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

85. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

86. The first and second components of the immunotherapeutic bioresponsive gel matrixes can be mixed prior to administration for simultaneous administration or kept separate for separate administration including separate topical administration (for example two separate sprays) or mixed during concurrent administration (for example a single applicator that mixes the separate components as application is occurring or allows for simultaneous or concurrent application).

87. Compositions for oral administration include powders or granules, suspensions or solutions in water or non-aqueous media, capsules, sachets, or tablets. Thickeners, flavorings, diluents, emulsifiers, dispersing aids or binders may be desirable..

88. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

89. Effective dosages and schedules for administering the compositions may be determined empirically, and making such determinations is within the skill in the art. The dosage ranges for the administration of the compositions are those large enough to produce the desired effect in which the symptoms of the disorder are effected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above.

### C. Method of treating cancer

90. In one aspect, it is understood that the therapeutic agent delivery vehicles disclosed herein are intended for administration to a subject to treat, prevent, inhibit, or reduce a cancer or metastasis or to treat, prevent, inhibit, or reduce a relapse or metastasis following surgical recision (i.e., resection). Thus, disclosed herein are methods of treating, inhibiting, and/or preventing tumor proliferation and/or metastasis at the site of surgical resection of a cancer tumor comprising administering to a subject at the site of the resection, an immunotherapeutic bioresponsive gel matrix comprising a first component and a second component; wherein gelation of the matrix occurs when the first component comes into contact with the second component; and wherein at least one of the components comprises at least one first nanoparticle; wherein the at least one first nanoparticle comprises at least one anti-cancer agent, blockade inhibitor, or immunomodulatory agent. Thus, in one aspect, disclosed herein are methods of treating, inhibiting, and/or preventing tumor proliferation and/or metastasis at the site of surgical resection of a cancer tumor comprising administering to a subject at the site of the resection any of the immunotherapeutic bioresponsive gel matrix disclosed herein.

91. As noted herein, the disclosed immunotherapeutic bioresponsive gel matrixes can be used to treat any disease where uncontrolled cellular proliferation occurs such as cancers. A representative but non-limiting list of cancers that the disclosed compositions can be used to treat is the following: leukema (including, but not limited to, acute myeloid leukemia (AML), chronic myeloid leukemia, acute lymphoblastic leukemia (ALL), lymphoma; B cell lymphoma; T cell lymphoma; mycosis fungoides; Hodgkin's Disease; leukemias, including but not limited to myeloid leukemia; plasmacytomas; histiocytomas; bladder cancer; brain cancer, nervous system cancer, head and neck cancer, squamous cell carcinoma of head and neck, urothelial cancer, kidney cancer, lung cancers such as small cell lung cancer and non-small cell lung cancer, neuroblastoma, glioblastoma, ovarian cancer, pancreatic cancer, prostate cancer, skin cancer, liver cancer, melanoma, squamous cell carcinomas of the mouth, throat, larynx, and lung; colon cancer; cervical cancer; cervical carcinoma; breast cancer; epithelial cancer; renal cancer, genitourinary cancer; pulmonary cancer; esophageal carcinoma; head and neck carcinoma; large bowel cancer; hematopoietic cancers; testicular cancer; colon and rectal cancers; prostatic cancer; AIDS-related lymphomas or sarcomas, metastatic cancers, or cancers in general; or pancreatic cancer.

92. Accordingly, in one aspect, disclosed herein are methods of treating, reducing, inhibiting, or preventing a cancer (including, but not limited to acute myeloid leukemia (AML), chronic myeloid leukemia, acute lymphoblastic leukemia (ALL), non-Hodgkin's lymphoma (NHL), multiple myeloma (MM), melanoma, renal cell carcinoma, urothelial carcinoma, non-small cell lung carcinoma, and/or bladder cancer); proliferation of a cancer (including, but not limited to acute myeloid leukemia (AML), chronic myeloid leukemia, acute lymphoblastic leukemia (ALL), non-Hodgkin's lymphoma (NHL), multiple myeloma (MM), melanoma, renal cell carcinoma, urothelial carcinoma, non-small cell lung carcinoma, and/or bladder cancer); metastasis of a cancer (including, but not limited to acute myeloid leukemia (AML), chronic myeloid leukemia, acute lymphoblastic leukemia (ALL), non-Hodgkin's lymphoma (NHL), multiple myeloma (MM), melanoma, renal cell carcinoma, urothelial carcinoma, non-small cell lung carcinoma, and/or bladder cancer); and/or treating, reducing, inhibiting, or preventing relapse, proliferation or metastasis of a cancer following surgical recision of a tumor (including, but not limited to acute myeloid leukemia (AML), chronic myeloid leukemia, acute lymphoblastic leukemia (ALL), non-Hodgkin's lymphoma (NHL), multiple myeloma (MM), melanoma, renal cell carcinoma, urothelial carcinoma, non-small cell lung carcinoma, and/or bladder cancer) in a subject comprising administering to a patient with a cancer the immunotherapeutic bioresponsive gel matirxes disclosed herein. Thus, in one aspect, disclosed herein are methods of treating, reducing, inhibiting, or preventing a cancer; proliferation of a cancer; and/or treating, reducing, inhibiting, or preventing relapse, proliferation or metastasis of a cancer following surgical resection of a tumor in a subject comprising administering to a subject a composition comprising any of the bioresponsive hydrogel matrixes disclosed herein (including for example, a bioresponsive hydrogel matrix comprising an anti-CD47 antibody).

93. As noted above, the immunotherapeutic bioresponsive gel matrixes used in the disclosed methods of treating, inhibiting, and/or preventing tumor proliferation and/or metastasis at the site of surgical resection of a cancer comprise a therapeutic agent cargo such as, for example an anti-cancer agent, blockade inhibitor, or immunomodulatory agent. For example, the therapeutic agent cargo can comprise an immune checkpoint inhibitor such as for example, a PD-1 inhibitor, a PD-L1 inhibitor, or CTLA-4 inhibitor (such as, for example, nivolumab, pembrolizumab, pidilizumab, BMS-936559, Atezolizumab, Durvalumab, or Avelumab); an immunomodulatory agent such as, for example, any antibody, cytokine, chemokine, nucleic acid, or small molecule the inhibits or promotes the activity, production, proliferation, or maturation of immune cells including but not limited to NK cells, NK T cells, T cells, B cells, plasma cells, macrophage, dendritic cells, and mast cells. In one aspect, the immunomudulator agent can be IFN-γ, TNF-α, TGF-β, IL-1β, IL-2 IL-4, IL-5, IL-6, IL-10, IL-12, anti-CD3 antibody, anti-CD4 antibody, anti-CD8 anti-body, anti-CTLA-4 antibody, anti-4-1BB antibody, anti-OX40 antibody, anti-CD25 antibody, anti-CD27 antibody, anti-LAG3 antibody, and/or anti-CD47 antibody

94. In one aspect, the bioresponsive gel matrix used in the disclosed methods of treating a cancer in a subject comprises a anti-cancer agent. The anti-cancer agent used in the disclosed methods can comprise any chemotherapeutic known in the art, the including, but not limited to Abemaciclib, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin Chlorambucil), Amifostine, Aminolevulinic Acid, Anastrozole, Aprepitant, Aredia (Pamidronate Disodium), Arimidex (Anastrozole), Aromasin (Exemestane),Arranon (Nelarabine), Arsenic Trioxide, Arzerra (Ofatumumab), Asparaginase Erwinia chrysanthemi, Atezolizumab, Avastin (Bevacizumab), Avelumab, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin) , Bevacizumab, Bexarotene, Bexxar (Tositumomab and Iodine I 131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Campath (Alemtuzumab), Camptosar, (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride , EPOCH, Erbitux (Cetuximab), Eribulin Mesylate, Erivedge (Vismodegib), Erlotinib Hydrochloride, Erwinaze (Asparaginase Erwinia chrysanthemi) , Ethyol (Amifostine), Etopophos (Etoposide Phosphate), Etoposide, Etoposide Phosphate, Evacet (Doxorubicin Hydrochloride Liposome), Everolimus, Evista , (Raloxifene Hydrochloride), Evomela (Melphalan Hydrochloride), Exemestane, 5-FU (Fluorouracil Injection), 5-FU (Fluorouracil-Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil--Topical), Fluorouracil Injection, Fluorouracil--Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, GEMCITABINE-CISPLATIN, GEMCITABINE-OXALIPLATIN, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride) , Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride, Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and , Hyaluronidase Human, ,Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq , (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil--Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Vandetanib, VAMP, Varubi (Rolapitant Hydrochloride), Vectibix (Panitumumab), VeIP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), and/or Zytiga (Abiraterone Acetate).

95. It is understood and herein contemplated that the hydrogel matrix can be designed to be bioresponsive to the microenvironment of the tumor and release the anti-cancer agent, blockade inhibitor, or immunomodulatory agent upon exposure to factors within the microenvironment such as, for example reactive oxygen species or pH. The matrix can be designed to alter the pH of the TME to modulate the release rate of the anti-cancer agent, blockade inhibitor, or immunomodulatory agent. For example, the bioresponsive matrix can comprise a H+ scavenger such as, for example, fibrinin which will raise the pH of the microenvironment and result in prolonged release. In one aspect, it is contemplated herein that the bioresponsive gel matrix can be designed to release the anti-cancer agent, blockade inhibitor, or immunomodulatory agent into the tumor microenvironment for at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42,4 3,44, 45, 46, 47, 48, 49, 50, 51, 52, 53, 54, 55, 56, 57, 58, 59, 60, 65, 70, 75, 80, 85, or 90 days.

96. "Treat," "treating," "treatment," and grammatical variations thereof as used herein, include the administration of a composition with the intent or purpose of partially or completely preventing, delaying, curing, healing, alleviating, relieving, altering, remedying, ameliorating, improving, stabilizing, mitigating, and/or reducing the intensity or frequency of one or more a diseases or conditions, a symptom of a disease or condition, or an underlying cause of a disease or condition. Treatments described here may be applied preventively, prophylactically, pallatively or remedially. Prophylactic treatments are administered to a subject prior to onset (e.g., before obvious signs of cancer), during early onset (e.g., upon initial signs and symptoms of cancer), or after an established development of cancer. Prophylactic administration can occur for day(s) to years prior to the manifestation of symptoms of an infection.

97. As described here, the disclosed methods of treating, preventing, inhibiting, or reducing a cancer or metastasis or to treating, preventing, inhibiting, or reducing a relapse or metastasis following surgical recision (i.e., resection) comprising administering to a subject any of therapeutic agent delivery vehicles or pharmaceutical compositions disclosed herein can comprise administration of the therapeutic agent delivery vehicles or pharmaceutical compositions at any frequency appropriate for the treatment of the particular cancer in the subject. For example, therapeutic agent delivery vehicles or pharmaceutical compositions can be administered to the patient at least once every 12, 14, 16, 18, 20, 22, 24, 26, 28, 30, 32, 34, 36, 38, 40, 42, 44, 46, 48 hours, once every 3, 4, 5, 6, 7, 8, 9, 10, 11, 12, 13, 14, 15, 16, 17, 18, 19, 20, 21, 22, 23, 24, 25, 26, 27, 28, 29, 30, 31 days, once every 2, 3, 4, 5, 6, 7, 8, 9, 10, 11, or 12 months. In one aspect, the therapeutic agent delivery vehicles or pharmaceutical compositions are administered at least 1, 2, 3, 4, 5, 6, 7 times per week.

98. It is understood and herein contemplated that the first and second component that when placed in contact for the immunotherapeutic bioresponsive gel matrix can be mixed prior to administration to form the gel matrix and administered simultaneously or administered separately and allowed to form the gel matrix at the site of administration. Thus, it is contemplated herein that the first and second components of the immunotherapeutic bioresponsive gel matrix can be maintained separately and administered concurrently or sequentially. For example, the first component can be administered to the site of the tumor in the subject at least 1, 2, 3, 4, 5, 10, 15, 20, 25, 30, 35, 40, 45, 50, 55 minutes, 1, 2, 3, 4, 5, 6, 7, 8, 9, 10, 11, 12, 18, 24, 36, 48, or 72 hours before the second component. Similarly, the first component can be administered to the site of the tumor in the subject at least 1, 2, 3, 4, 5, 10, 15, 20, 25, 30, 35, 40, 45, 50, 55 minutes, 1, 2, 3, 4, 5, 6, 7, 8, 9, 10, 11, 12, 18, 24, 36, 48, or 72 hours after the second component.

99. It is understood and herein contemplated that there can be benefits to preventing metastasis or post-resection proliferation of a tumor that can be achieved by administering the immunotherapeutic bioresponsive gel matrix prior to resection. Thus, in one aspect, disclosed herein are methods of treating, preventing, inhibiting, or reducing a cancer or metastasis or to treating, preventing, inhibiting, or reducing a relapse or metastasis following surgical recision (i.e., resection), wherein at least one of the first and second component is administered to the site of resection prior to the removal of the tumor.

100. Tumor associated macrophages (TAMs) usually constitute a substantial percentage of tumor infiltrating immune cells. TAMs differentiate into diverse functional phenotypes, denoted classically activated (also called M1-like phenotype) macrophages and alternatively activated (also called M2-like phenotype) macrophages. The M1 type TAMs overexpress essential major histocompatibility complex class I and class II molecules for antigen presentation, which plays critical roles in antitumor immunity. Conversely, M2 type TAMs exert pro-tumorigenic activities. Progressive tumors typically perform M2 type TAMs activation responding to different tumor determining factors, especially the low pH value. M2-polarized TAMs infiltration in the tumor is usually related to poor outcome in clinic, as it generally promotes the tumor invasion and migration and angiogenesis, and also suppresses T cell responses. Thus, M2 polarization of TAMs in the particular TME seems to be a critical adverse prognostic factor for cancer treatment.

101. Herein, a sprayed bioresponsive immunotherapeutic fibrin gel was engineered to inhibit tumor recurrence and metastasis after surgery. The fibrin gel, an FDA approved material, was formed *via* the fibrinogen and thrombin interaction. The fibrinogen solution containing CaCO₃ nanoparticles (NPs) pre-encapsulated with anti-CD47 antibody (aCD47@CaCO₃) and the thrombin solution were quickly sprayed at the tumor resection site post-surgery and immediately formed an *in situ* immunotherapeutic fibrin gel (Figure 1a). CaCO₃ NPs are expected to gradually dissolve and subsequently release the encapsulated aCD47 in the acidic inflammation and tumor microenvironment (TME), which usually plays an immunosuppressive role by affecting immune cells functions including TAM polarization, T-cell proliferation, and cytokine production. It was hypothesized that CaCO₃ NPs could relieve adverse effects of the acidic environment, such as promoting the activation of M1 type TAMs.

102. Meanwhile, the released anti-CD47 antibody can block the interaction between CD47 and SIRP*α*, increasing macrophage phagocytosis of cancer cells and tumor antigen presentation to T cells, thus activating the adaptive immune response. The results demonstrated that the clinically approved fibrin gel containing aCD47@CaCO₃ NPs can effectively prevent tumor recurrence and inhibit metastasis after surgery.

103. Also disclosed herein are methods of increasing or modulating the innate or adaptive immune response to a tumor following resection of the tumor comprising administering to a subject with a tumor an immunotherapeutic bioresponsive ge1 matrix comprising a first component and a second component; wherein gelation of the matrix occurs when the first component comes into contact with the second component; and wherein at least one of the components comprises at least one first nanoparticle; wherein the at least one first nanoparticle comprises at least one anti-cancer agent, blockade inhibitor, or immunomodulatory agent. For example, disclosed herein are methods of modulating the innate immune response such that macrophage conform to the M1 type comprising administering the disclosed immunotherapeutic bioresponsive agents.

104. It is understood and herein contemplated that disclosed bioresponsive gels can have systemic effects despite being administered locally. Thus, in one aspect, disclosed herein of methods of treating, reducing, inhibiting, and/or preventing a cancer in a subject; wherein administration of the immunotherapeutic bioresponsive gel matrix increases the anti-tumor response and slows tumor proliferation systemically in the subject.

105. In one aspect, the amount of the therapeutic agent, or pharmaceutical compositions disclosed herein which are administered to the subject for use in the disclosed methods can comprise any amount appropriate for the treatment of the subject for the particular cancer as determined by a physician. For example, the amount of the therapeutic agent delivery vehicles, or pharmaceutical composition can be from about 10mg/kg to about 100mg/kg. For example, the amount of the therapeutic agent delivery vehicles, or pharmaceutical composition administered can be at least 10mg/k, 11mg/kg, 12mg/kg, 13mg/kg, 14mg/kg, 15mg/kg, 16mg/kg, 17mg/kg, 18mg/kg, 19mg/kg, 20mg/kg, 21mg/kg, 22mg/kg, 23mg/kg, 24mg/kg, 25mg/kg, 30mg/kg, 35mg/kg, 40mg/kg, 45mg/kg, 50mg/kg, 55mg/kg, 60mg/kg, 65mg/kg, 70mg/kg, 75mg/kg, 80mg/kg, 85mg/kg, 90mg/kg, 95mg/kg, or 100mg/kg. Accordingly, in one aspect, disclosed herein are methods of treating a cancer in a subject, wherein the dose of the administered therapeutic agent delivery vehicles, or pharmaceutical composition is from about 10mg/kg to about 100mg/kg.

### D. Examples

106. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

### a) Results

### (1) Formation of fibrin gel containing aCD47@CaCOs NPs by quick spray

107. The aCD47@CaCO₃ NPs with the loading capacity of 5% and encapsulation efficiency of 50% were precipitated in the solution containing poly(ethylene glycol)-b-poly(glutamic acid) (PEG-b-P(Glu)) block copolymers by the addition of Ca²⁺ and CO₃²⁻. Monodisperse aCD47@CaCO₃ NPs with a diameter about 100 nm were obtained, the size of which was controlled by PEG-b-P(Glu) *via* the interaction between carboxyl and Ca²⁺, with a PEG shell to eliminate further aggregation and agglomeration (Figure 1b, 1c, and 1d). The elemental mapping images (Figure 1c) indicated the homogenous distribution of aCD47 (gadolinium-chelated) in those CaCO₃ NPs.

108. Fibrin gel can be quickly formed by simultaneously spraying and mixing equal volumes of solutions containing either fibrinogen with aCD47@CaCO₃ NPs or thrombin, which was validated by the rheology test (Figures 2A, 2B, and 2C). Ca²⁺ in the solution associated with CaCO₃ NPs could facilitate the formation of fibrin gel. The mixture of two solutions resulted in a rapid increase in the elastic modulus (G). The morphology of the fibrin gel containing aCD47@CaCO₃ NPs was observed by cryo-scanning electron microscopy (Cryo-SEM) imaging (Figures 1e and1f). To further substantiate the distribution of aCD47@CaCO₃ NPs in the hydrogel, fluorescein (FITC) labeled fibrinogen and Cy5.5 labeled CaCO₃ NPs were used for hydrogel formation. The frozen sections were then stained with the Rhodamine-conjugated donkey anti-rat IgG antibody to track aCD47. As demonstrated by the confocal imaging, Cy5.5 signals from CaCO₃ exhibited a uniform distribution pattern, which were co-localized with aCD47, further indicating the encapsulation of aCD47 in the CaCO₃ NPs (Figure 1g). The biodegradation behavior of Cy5.5 labeled fibrin gel was monitored by fluorescence *in vivo* imaging system. Three weeks after spraying, the fluorescence signal from the gel was undetectable, indicating the complete degradation of the gel.

109. CaCO₃ NPs can be dissolved and released the encapsulated aCD47 by reacting with H⁺ in the acidic buffer. Meanwhile, acting as a proton scavenger, CaCO₃ NPs can also elevate the pH values of the acidic solution (Figures 3 and 4). The biodegradation behavior of Cy5.5 labeled fibrin gel was monitored by fluorescence *in vivo* imaging system. Three weeks after spraying, the fluorescence signal from the gel was undetectable, indicating the complete degradation of the gel.

110. CaCO₃ NPs dissolved and released the encapsulated aCD47 by reacting with H⁺ in the acidic buffer (Fig. 3A, Fig. 3B, and Fig. 4). Meanwhile, CaCO₃ NPs elevated the pH values of the acidic solution as a proton scavenger (Fig. 5). Encapsulation of aCD47@CaCO₃ NPs in the hydrogel allowed gradual release of aCD47 (Figure 1h). To evaluate the release behavior *in vivo,* aCD47 was either dispersed in phosphate buffered saline (PBS), encapsulated in CaCO₃ NPs, or in CaCO₃@Fibrin gel, and then injected or spayed within the tumor resection cavity. The Cy5.5 labeled aCD47 signal was monitored and quantified by an *in vivo* imaging system (IVIS) (Figure 1i, FIG. 1j, and Fig. 6). While 80% of the signal from the injected antibody in solution was undetectable at the resection site, 79% of aCD47 released from the CaCO₃@Fibrin gel remained detectable. The sustained release of aCD47 was further substantiated by confocal imaging of residual tumor sections, in which remarkable fluorescence signal associated with the antibodies was observed away from NPs' signal, indicating degradation of the CaCO₃ NPs and sustained release of the antibodies from the NPs. Although the sustained release of therapeutics from gel-nanoparticle reservoir is advantageous compared with the free cargo, the release kinetics desired for this application needs further optimization to maximize the therapeutic effects and minimize toxicities.

### (2) Immune response induced by CaCO₃ NPs and aCD47

111. Glycolytic metabolism of cancer cells in the hypoxia TME leads to the production of lactate and H⁺ (pH=6.5-6.8), regulating the roles and functions of immune cells inside tumors. Besides, the local acidification (pH=6.0-7.0) is also found at the inflammation and tissue injury site. Considering the ability of CaCO₃ to eliminate the H⁺ in the residual TME and inflammation environment, the overall immune effects were examined after CaCO₃@Fibrin gel treatment. A significant reduction of M2-TAMs (CD206^{hi}CD11b⁺F4/80⁺) and an increase of M1-TAMs (CD80^{hi}CD11b⁺F4/80⁺) were found after CaCO₃@Fibrin treatment, indicating the promoted polarization of TAMs to the tumor inhibiting M1 phenotype (Figure 7a and 7b). This polarization of TAMs was further confirmed by the level variation of cytokines in the TME (Figure 7c). The observed polarization of macrophages to M1 phenotype could be ascribed to the modulation of the acidity of the TME that usually favors M2-like macrophage polarization. Compared to small molecules that promote M1-like macrophage polarization, CaCO₃ scavenging of H⁺ within the inflamed tumor resection provides simpler approach. A correlation was observed between the concentration of CaCO₃ NPs and macrophage polarization as assessed by flow cytometry. After CaCO₃@Fibrin treatment, reduction of myeloid-derived suppressor cells (MDSCs) and regulatory T cells (Tregs) and reduced expression of HIF1-*a* in the TME were also observed (Figure 7d, 7e, 7f, and 7g; Fig. 8). Remarkably, CaCO₃@Fibrin treatment caused the increase of tumor-infiltrating lymphocytes (TILs, CD3⁺), especially cytotoxic T lymphocytes (CTLs, CD3⁺CD8⁺), within the tumor (Figure 7i) and the increase of interferon-y (IFN-y) and tumor necrosis factor-*α* (TNF-*α*) in the plasma (Figure 7h). Meanwhile, the expression of programmed death-1 (PD-1) and programmed death-ligand 1 (PD-L1) on immune cells and cancer cells, respectively, remained nearly unchanged or slightly decreased (Fig. 9A and 9B).

112. CD47 blockade increased the phagocytosis of cancer cells by macrophages *in vitro,* as shown in the confocal imaging and flow cytometry results (Fig. 3a, Fig. 3b, Fig. 11a, and Fig. 11b). When aCD47 was loaded into the fibrin gel (aCD47: 50 µg per mouse) and sprayed at the tumor resection site, increased frequency of Ly6C^{hi}Ly6G⁻ macrophages within the resection tumor cavity was observed, but not for Ly6G⁺Ly6C^{dim} neutrophils (Fig. 3c, Fig. 3d, and Fig. 12). Increase of CD11c⁺ DCs was also recorded, and these cells showed expression of CD80, CD86 and CD103 denoting their maturation status (Fig. 3e and Fig. 3f). Thus, CD47 blockade enhances phagocytosis of cancer cells by both macrophages and DCs, activating innate immune systems.

### (3) aCD47@CaCO₃@Fibrin therapy for inhibition of tumor recurrence

113. To validate the therapeutic efficacy of aCD47@CaCO₃@Fibrin on residual microtumors after surgery, incomplete tumor resection in a B16F10 mouse model was established to mimic tumor recurrence after surgery. The *in situ*-formed fibrin containing IgG@CaCO₃, or aCD47, or aCD47@CaCO₃ (aCD47: 50 µg per mouse) was sprayed at the resection site. The recurrence of the tumors was monitored by the bioluminescence signals from B16F10 cells (Figure 13a). Mice in the group after aCD47@CaCO₃@Fibrin treatment showed the smallest relapsed tumor size, with four out of eight mice in this group exhibiting no detectable tumor (Fig. 13b and 13c). In contrast, mice treated with IgG@CaCO₃@Fibrin or aCD47@Fibrin showed modestly delayed tumor growth, and only one out of six mice without tumor recurrence. Compared with the untreated control group, the bland fibrin gel showed a negligible therapeutic effect (Figure 13b and 13c). The survival of the mice correlated with the tumor size was monitored. Fifty percent of the mice after aCD47@CaCO₃@Fibrin treatment survived in the observation period of 60 days. In contrast, only two mice survived in all control groups after two months (Figure 13d). Body weights of mice were not obviously affected after different treatments (Figure 13e). Moreover, histology analysis of major organs collected from mice 30 days after aCD47@CaCO₃@Fibrin treatment, together with the complete blood panel test and serum biochemistry assay conducted at 1, 7, and 14 days after treatment indicated that local delivery of aCD47 would not induce significant side effects to mice

114. To dissect the changes among immune cells, the relapsed-tumors were collected and analyzed by flow cytometry and immunofluorescence staining five days after surgery. Both the innate and adaptive immune systems were focused on, particularly macrophages and TILs. The proportion of macrophages in the residual tumor was increased after aCD47 blockade, together with an increased polarization of M1 phenotype TAMs after CaCO₃ treatment (Fig. 14a; Fig. 15a, Fig. 15b, Fig. 15c, and Fig. 15d). Similarly, the absolute number of TILs (CD3⁺ cells) was also increased in the residual tumor after aCD47@CaCO₃@Fibrin treatment (Figure 14b). Furthermore, the percentage of CD8⁺ T cells significantly was increased in the tumors after aCD47@CaCO₃@Fibrin treatment versus all other controls (Figure 14b-c). Compared with untreated or blank fibrin treated controls, proliferation of CD4⁺ T cells also increased, while the percentage of regulatory T cells (CD4⁺Foxp3⁺ T cells) decreased (Fig. 14d, Fig. 16a, and Fig. 16b). Immunofluorescence staining visually indicated the marked increased macrophages and CD8⁺ T cells in the residual tumors after aCD47@CaCO₃@Fibrin therapy (Fig. 14e, Fig. 17a, and Fig. 17b). Secretion of cytokines, an important indicator during the process of immune responses, further confirmed the effective innate and adaptive immune responses induced by aCD47@CaCO₃@Fibrin (Figure 14f).

### (4) aCD47@CaCO₃@Fibrin therapy for treating distant tumor

115. With the confirmation that aCD47@CaCO₃@Fibrin can activate the innate and adaptive immune cells in the residual tumor, it was sought to investigate whether the local treatment of aCD47@CaCO₃@Fibrin can trigger the systemic immune responses. B16F10 cancer cells were inoculated on the opposite side of the primary tumor on each mouse to mimic the metastatic tumors. Then the primary tumors were partially resected, and fibrin gel containing aCD47@CaCO₃ NPs was sprayed at the tumor resection site (Figure 18a). Tumor burden was monitored according to the bioluminescence signals from B16F10 cells by an IVIS imaging system. It was found that the local tumor recurrence was inhibited effectively *via* aCD47@CaCO₃@Fibrin treatment, and the distant tumors on the opposite side without any extra treatment also showed a slow growth rate (Figures 18b, 18c, and 18d). Consistent with these results, the number of M1-like TAMs and CD103⁺ DCs were significantly increased in tumors sprayed with aCD47@CaCO₃@Fibrin (Figures 18e and 18f), and CD8⁺ T cells were increased in both treated and distant tumors (Figure 18g & Fig. 19). The increased CD8⁺ T cells in the distant tumor can be attributed to local cross-presentation of tumor antigens by macrophages and DCs that trigger systemic antitumor immunity. The activation of immune system was further confirmed by the peritumoral injection of aCD47@CaCO₃@Fibrin by a dual-syringe administration method. aCD47@CaCO₃@Fibrin-mediated treatment inhibited the growth of both local and distant tumor effectively.

116. The combination efficacy of anti-PD-1 (aPD1) and aCD47 co-delivered by sprayed gel was also evaluated. Similarly, the primary tumors were partially resected and the surgical sites were sprayed with fibrin gel containing aCD47@CaCO₃ NPs (aCD47: 50 µg per mouse), aPD1@CaCO₃ NPs (aPD1: 50 µg per mouse) or aCD47&aPD1@CaCO₃ NPs (aCD47: 25 µg per mouse, aPD1: 25 µg per mouse). Encouragingly, a synergistic effect was achieved by aCD47&aPD1@CaCO₃@Fibrin treatment in inhibiting tumor recurrence and metastasis as shown in the bioluminescence imaging and the growth profiles of the local and distant tumors Figures 20A, 20B, and 20C).

### b) Conclusion

In conclusion, the immunotherapeutic fibrin spayed at the tumor resection site can reverse the immunosuppressive TME and induce systemic antitumor immunological responses to prevent the local cancer recurrence and restrain existing metastasis. CaCO₃ NPs can modulate the acidic tumor resection environment associated with the residual TME and inflammation environment by eliminating H⁺ in a bioresponsive manner, thereby effectively promoting the antitumor immune responses. Along with this reaction, the released aCD47 from CaCO₃ NPs can block the up-regulated "don't eat me" signal on cancer cells, allowing programmed cancer cell removal by macrophages. CD47 blockade can also lead to T cell-mediated destruction of cancer cells owing to the enhanced presentation of tumor-specific antigen by macrophages and DCs.

### c) Materials and Methods

### (1) Materials, cell lines, and animals.

117. All chemicals were purchased from Sigma-Aldrich without any purification. Mouse thrombin and fibrinogen were purchased from Molecular Innovations. Anti-CD47 antibody (aCD47) was purchased from Biolegend Inc (Cat. # 127518, Clone: miap301). Mouse melanoma (B16F10) cells were purchased from the American Type Culture Collection (ATCC). B16F10-luc cells were obtained from Dr. Leaf Huang at UNC. The cells were maintained in Dulbecco's Modified Eagle Medium (Gibco, Invitrogen) with 10% fetal bovine serum (Invitrogen, Carlsbad, CA) and 100 U/mL penicillin (Invitrogen) in an incubator (Thermo Scientific) at 37 °C under an atmosphere of 5% CO₂. Female C57BL/6 mice (6-10 weeks) were purchased from Jackson Lab. All mouse studies were performed following the protocols approved by the Institutional Animal Care and Use Committee at the University of North Carolina at Chapel Hill and North Carolina State University.

### (2) Preparation and characterization of aCD47@CaCOs.

118. aCD47@CaCO₃ NPs were prepared by chemical precipitation. Typically, 1 ml of Tris-HCl buffer (1 mM, pH 7.6) containing 100 mM CaCl₂ was firstly mixed with 1 ml HEPES saline buffer (50 mM, pH 7.1, NaCl 140 mM) that contained 100 µg aCD47, 10 mg PEG-b-P(Glu) block copolymers that were synthesized, and 10 mM NaCO₃. Then, the mixture was stirred overnight at 4 ^{O}C. The excess ions, copolymers and antibodies were removed by centrifugation at 14 800 rpm for 10 min. The size distribution was evaluated by DLS and the morphology was measured by TEM (JEOL 2000FX). The elemental analysis of aCD47@CaCO₃ (aCD47 was chelated gadolinium) was characterized using an analytical TEM (Titan). The amount of aCD47 encapsulated in CaCO₃ was measured by ELISA (rat IgG total ELISA kit, eBioscience, cat. no. 88-50490-22). The loading capacity (LC) and encapsulation efficiency (EE) of CaCO₃ NPs were calculated to the following formula: LC= (A-B)/C and EE= (A - B)/A, where A was the expected encapsulated amount of antibody, B was the free non-entrapped antibody, and C was the total weight of particles.

### (3) Formation and characterization of

### aCD47@CaCO₃@Fibrin.

119. Fibrin gels were obtained by spraying equal volumes of fibrinogen (10 mg/ml) containing aCD47@CaCO₃ and thrombin (5 NIH U/mL). The morphology of aCD47@CaCO₃@Fibrin was characterized by Cryo-SEM (JEOL 7600F with Gatan Alto). The encapsulation of aCD47@CaCO₃ in fibrin was further characterized by a confocal microscope (Zeiss LSM 710).

### (4) Release of aCD47 in vitro.

120. The release behavior of aCD47 was studied at 37 ^{O}C in phosphate buffered saline (PBS) at different pH values (pH 6.5 and 7.4). The released aCD47 was measured by a Rat IgG total ELISA kit.

### (5) Release of aCD47 in vivo.

121. To study the *in vivo* release behavior of aCD47, Cy5.5-labeled free aCD47 or aCD47@CaCO₃ dispersed in PBS was injected at the tumor resection site. Moreover, equal volumes of fibrinogen solution (10 mg/ml) containing aCD47@CaCO₃ and thrombin (5 NIH U/mL) were spayed at the tumor site after surgery. Fluorescence imaging was monitored by an IVIS Spectrum system (Perkin Elmer).

### (6) In vitro phagocytosis assay.

122. Bone marrow derived macrophages (BMDMs) were labeled with CellTracker Green (C7025, Thermo-Fisher Scientific), and cancer cells were labeled with CellTracker DeepRed (C34565, Thermo-Fisher Scientific, USA). Macrophages (1 × 10⁵) were co-cultured with cancer cells (4 × 10⁵) pre-blocked with IgG or aCD47 in the serum free medium. After incubation for 2 h at 37 ^{O}C, the phagocytosis was studied by confocal microscopy (Zeiss LSM 710) and CytoFLEX flow cytometry (Beckman).

### (7) In vivo tumor models and treatment.

123. To study the therapeutic effects on tumor recurrence, 10 days after 1× 10⁶ fLuc-B16F10 were transplanted into the right flank of mice, the mice were randomly divided into five groups (n=6-8) and their tumors were resected, leaving ~1% residual tumor tissues behind to mimic the residual microtumors after surgery. Briefly, animals were anesthetized in an induction chamber using isoflurane (up to 5% for induction; 1-3% for maintenance), and anaesthesia was maintained *via* a nose cone. The tumor area was clipped and aseptically prepped. Sterile instruments were used to remove approximately 99% of the tumor. Immediately after surgery, fibrin gels with different formations, including Fibrin, IgG@CaCO₃@Fibrin, aCD47@Fibrin, and aCD47@CaCO₃@Fibrin with the same dose were sprayed on the surgical bed. Then, the wound was closed by an Autoclip wound clip system.

124. For the metastatic tumor model, 7 days after 1 × 10⁶ fLuc-B16F10 were transplanted into the right flank of mice, a second tumor as the metastatic tumor (1 × 10⁶ fLuc-B16F10) was inoculated into the left flank of each mouse. Three days later, their primary tumors were resected, leaving ~1% residual tumor tissues behind to mimic the residual microtumors after surgery as described above. Similarly, aCD47@CaCO₃@Fibrin with the same dose were sprayed on the surgical bed after surgery.

125. The tumor size was measured by a caliper and calculated according to the following formula: width² × length × 0.5. The tumor was also monitored by the bioluminescence imaging. 10 min after intraperitoneal injection of d-luciferin (Thermo Scientific^{™} Pierce^{™}) in DPBS (15 mg/mL) into the mice (10 µL/g of body weight), mice were imaged *via* an IVIS Spectrum Imaging System (Perkin Elmer Ltd) for 5 min. Regions of interest were quantified as the average radiance (photons s⁻¹ cm⁻² sr⁻¹) using the Living Image software. Animals were euthanized with carbon dioxide when exhibiting signs of impaired health or when the volume of the tumor exceeded 1.5 cm³.

### (8) Cytokine detection.

126. The local and plasma levels of IL10, IL12p70, IL 6, IFN-γ, or TNF-α were measured with ELISA kits according to vendors' protocols (eBioscience). For detection of the local (tumor) concentration of cytokines, 5 days after spraying the fibrin gels, thetumor tissue was collected and homogenized in cold PBS to form single cell suspensions. For the plasma levels, serum samples were isolated from mice after different treatments and diluted for analysis.

### (9) Flow cytometry.

127. To study the immune cells within tumor, tumors collected from mice in different groups were cut into small pieces and homogenized in cold staining buffer to form single cell suspensions. Then, the single cell suspension was prepared by gentle pressure with the homogenizer without the addition of digestive enzyme. Cells were stained with fluorescence-labeled antibodies CD45 (Biolegend, cat. no. 103108), CD11b (Biolegend, cat. no. 101208), CD206 (Biolegend, cat. no. 141716), F4/80 (Biolegend, cat. no. 123116), CD80 (Biolegend, cat. no. 104722), Gr-1 (Biolegend, cat. no. 108412), CD3 (Biolegend, cat. no. 100204), CD4 (Biolegend, cat. no. 100412), CD8 (Biolegend, cat. no. 140408), Foxp3 (Biolegend, cat. no. 126404), CD11c (Biolegend, cat. no. 117310), CD86 (Biolegend, cat. no. 105008), CD103 (Biolegend, cat. no. 121406), Ly6C (Biolegend, cat. no. 128016), and Ly6G (Biolegend, cat. no. 127608) following the manufacturers' instructions. All these antibodies used in the experiments were diluted 200 times. The stained cells were measured on a CytoFLEX flow cytometer (Beckman) and analyzed by the FlowJo software package (version 10.0.7; TreeStar, USA, 2014).

### (10)Immunofluorescence staining.

128. The tumors were dissected from the mice and snap frozen in optimal cutting temperature (OCT) medium. Tumor sections were cut using a cryotome and then mounted on slides. Then, the sections were stained with primary antibodies: CD4 (Abcam, cat. no. ab 183685), CD8 (Abcam, cat. no. ab22378), and F4/80 (Abcam, cat. no. ab100790) overnight at 4 °C following the manufacturers' instructions. Following the addition of fluorescently labelled secondary antibodies: goat anti-rat IgG (H+ L; Thermo Fisher Scientific, cat. no. A18866) and goat anti-rabbit IgG (H+L; Thermo Fisher Scientific, cat. no. A32733), the slides were analyzed using a confocal microscope (Zeiss LSM 710). All these antibodies used in the experiments were diluted 200 times.

### (11) Western blotting.

129. For Western blotting, each sample with the equal amount of protein measured by Bicinchoninic Acid Protein Assay Kit (BCA) was mixed with an equal volume of 2 × Laemmli buffer and was boiled at 95 ^{O}C for 5 min. After completion of gel electrophoresis, proteins were transferred to a Hybond nitrocellulose membrane. Then, anti-HIF1-α antibody at a dilution of 1:1000 (Santa Cruz, sc-13515), and anti-β-actin antibody (Abcam, ab8226) at a 1:5000 dilution were used as primary antibodies. The secondary antibody used for these blots was a goat antimouse antibody (Novus Biologicals, NBP175151).

### (12)Statistical analysis.

130. All results are appeared as the mean ± s.e.m. as indicated. Tukey post-hoc tests and One-way / two-way analysis of variance (ANOVA) were used for multiple comparisons (when more than two groups were compared). Survival benefit was determined using a log-rank test. All statistical analyses were performed using the Prism software package (PRISM 5.0; GraphPad Software, USA, 2007). The threshold for statistical significance was P < 0.05.

### E. References

Albain, K.S. et al. Radiotherapy plus chemotherapy with or without surgical resection for stage III non-small-cell lung cancer: a phase III randomised controlled trial. The Lancet 374, 379-386 (2009).
Andersen, M.H. The Balance Players of the Adaptive Immune System. Cancer Res. 78, 1379-1382 (2018).
Baer, C. et al. Suppression of microRNA activity amplifies IFN-γ-induced macrophage activation and promotes anti-tumor immunity. Nat. Cell Biol. 18, 790-802 (2016).
Baker, D., Masterson, T., Pace, R., Constable, W. & Wanebo, H. The influence of the surgical wound on local tumor recurrence. Surgery 106, 525-532 (1989).
Calandra, T. & Roger, T. Macrophage migration inhibitory factor: a regulator of innate immunity. Nat. Rev. Immunol. 3, 791-800 (2003).
Calcinotto, A. et al. Modulation of microenvironment acidity reverses anergy in human and murine tumor-infiltrating T lymphocytes. Cancer Res. 72, 2746-2756 (2012).
Carter, P.J. & Lazar, G.A. Next generation antibody drugs: pursuit of the'high-hanging fruit'. Nat. Rev. Drug Discov. 17, 197-223 (2018).
Chanmee, T., Ontong, P., Konno, K. & Itano, N. Tumor-associated macrophages as major players in the tumor microenvironment. Cancers 6, 1670-1690 (2014).
Chao, M.P. et al. Anti-CD47 antibody synergizes with rituximab to promote phagocytosis and eradicate non-Hodgkin lymphoma. Cell 142, 699-713 (2010).
Chen, Q. et al. A Self-Assembled Albumin-Based Nanoprobe for In Vivo Ratiometric Photoacoustic pH Imaging. Adv. Mater. 27, 6820-6827 (2015).
Chen, Q. et al. An albumin-based theranostic nano-agent for dual-modal imaging guided photothermal therapy to inhibit lymphatic metastasis of cancer post surgery. Biomaterials 35, 9355-9362 (2014).
Chen, Q. et al. Photothermal therapy with immune-adjuvant nanoparticles together with checkpoint blockade for effective cancer immunotherapy. Nat. Commun. 7, 13193 (2016).
Chen, Q. et al. Tumor vasculature normalization by orally fed erlotinib to modulate the tumor microenvironment for enhanced cancer nanomedicine and immunotherapy. Biomaterials 148, 69-80 (2017).
Coffey, J.C. et al. Excisional surgery for cancer cure: therapy at a cost. Lancet Oncol. 4, 760-768 (2003).
Colegio, O.R. et al. Functional polarization of tumor-associated macrophages by tumor-derived lactic acid. Nature 513, 559-563 (2014).
Credo, R., Curtis, C. & Lorand, L. Ca2+-related regulatory function of fibrinogen. Proc. Nat. Acad. Sci. 75, 4234-4237 (1978).
Edris, B. et al. Antibody therapy targeting the CD47 protein is effective in a model of aggressive metastatic leiomyosarcoma. Proc. Nat. Acad. Sci. 109, 6656-6661 (2012).
Gordon, S. Alternative activation of macrophages. Nat. Rev. Immunol. 3, 23-35 (2003).
Grivennikov, S.I., Greten, F.R. & Karin, M. Immunity, inflammation, and cancer. Cell 140, 883-899 (2010).
Herberman, R.R., Ortaldo, J.R. & Bonnard, G.D. Augmentation by interferon of human natural and antibody-dependent cell-mediated cytotoxicity. Nature 277, 221-223 (1979).
Huang, Y., Ma, Y., Gao, P. & Yao, Z. Targeting CD47: the achievements and concerns of current studies on cancer immunotherapy. J. Thorac. Dis. 9, E168-E174 (2017).
Jaiswal, S. et al. CD47 is upregulated on circulating hematopoietic stem cells and leukemia cells to avoid phagocytosis. Cell 138, 271-285 (2009).
Kershaw, M.H. & Smyth, M.J. Making Macrophages Eat Cancer. Science 341, 41-42 (2013).
Kwon, E.D. et al. Elimination of residual metastatic prostate cancer after surgery and adjunctive cytotoxic T lymphocyte-associated antigen 4 (CTLA-4) blockade immunotherapy. Proc. Nat. Acad. Sci. 96, 15074-15079 (1999).
Lawrence, T. & Natoli, G. Transcriptional regulation of macrophage polarization: enabling diversity with identity. Nat. Rev. Immunol. 11, 750-761 (2011).
Lee, E.J. et al. Nanocage-Therapeutics Prevailing Phagocytosis and Immunogenic Cell Death Awakens Immunity against Cancer. Adv. Mater. 30, 1705581 (2018).
Lewis, C.E., Harney, A.S. & Pollard, J.W. The multifaceted role of perivascular macrophages in tumors. Cancer Cell 30, 18-25 (2016).
Lin, E.Y. & Pollard, J.W. Tumor-associated macrophages press the angiogenic switch in breast cancer. Cancer Res. 67, 5064-5066 (2007).
Liu, Q. et al. Abrogation of local cancer recurrence after radiofrequency ablation by dendritic cell-based hyperthermic tumor vaccine. Mol. Ther. 17, 2049-2057 (2009).
Liu, Q. et al. Nanoparticle-Mediated Trapping of Wnt Family Member 5A in Tumor Microenvironments Enhances Immunotherapy for B-Raf Proto-Oncogene Mutant Melanoma. ACS Nano 12, 1250-1261 (2018).
Lu, Y., Aimetti, A. A., Langer, R. & Gu, Z. Bioresponsive materials. Nat. Rev. Mater. 2, 16075 (2017).
Mantovani, A., Sozzani, S., Locati, M., Allavena, P. & Sica, A. Macrophage polarization: tumor-associated macrophages as a paradigm for polarized M2 mononuclear phagocytes. Trends Immunol. 23, 549-555 (2002).
Martin, P. Wound healing--aiming for perfect skin regeneration. Science 276, 75-81 (1997).
Meyer, M.A. et al. Breast and pancreatic cancer interrupt IRF8-dependent dendritic cell development to overcome immune surveillance. Nat. Commun. 9, 1250 (2018).
Mi, P. et al. A pH-activatable nanoparticle with signal-amplification capabilities for non-invasive imaging of tumor malignancy. Nat. Nanotechnol. 11, 724-730 (2016).
Michaels, A.D. et al. CD47 Blockade as an Adjuvant Immunotherapy for Resectable Pancreatic Cancer. Clin. Cancer Res. 24, 1415-1425 (2018).
Neubert, N.J. et al. T cell-induced CSF1 promotes melanoma resistance to PD1 blockade. Sci. Transl. Med. 10, eaan3311 (2018).
Nishiyama, N. et al. Novel cisplatin-incorporated polymeric micelles can eradicate solid tumors in mice. Cancer Res. 63, 8977-8983 (2003).
Pardoll, D.M. The blockade of immune checkpoints in cancer immunotherapy. Nat. Rev. Cancer 12, 252-264 (2012).
Pyonteck, S.M. et al. CSF-1R inhibition alters macrophage polarization and blocks glioma progression. Nat. Med. 19, 1264-1272 (2013).
Ribas, A. & Wolchok, J.D. Cancer immunotherapy using checkpoint blockade. Science 359, 1350-1355 (2018).
Ring, N.G. et al. Anti-SIRPα antibody immunotherapy enhances neutrophil and macrophage antitumor activity. Proc. Nat. Acad. Sci. 114, 10578-10585 (2017).
Rodell, C.B. et al. TLR7/8-agonist-loaded nanoparticles promote the polarization of tumour-associated macrophages to enhance cancer immunotherapy. Nat. Biomed. Eng., 2, 578 (2018).
Rosenberg, S.A., Yang, J.C. & Restifo, N.P. Cancer immunotherapy: moving beyond current vaccines. Nat. Med. 10, 909-915 (2004).
Sockolosky, J.T. et al. Durable antitumor responses to CD47 blockade require adaptive immune stimulation. Proc. Nat. Acad. Sci. 113, 2646-2654 (2016).
Stephan, S.B. et al. Biopolymer implants enhance the efficacy of adoptive T-cell therapy. Nat. Biotechnol. 33, 97-101 (2015).
Tohme, S. et al. Neutrophil extracellular traps promote the development and progression of liver metastases after surgical stress. CancerRes. 76, 1367-1380 (2016).
Tumeh, P.C. et al. PD-1 blockade induces responses by inhibiting adaptive immune resistance. Nature 515, 568-571 (2014).
Turajlic, S. & Swanton, C. Metastasis as an evolutionary process. Science 352, 169-175 (2016).
Vakkila, J. & Lotze, M.T. Inflammation and necrosis promote tumor growth. Nat. Rev. Immunol. 4, 641-648 (2004).
Wang, C. et al. In situ activation of platelets with checkpoint inhibitors for post-surgical cancer immunotherapy. Nat. Biomed. Eng. 1, 0011 (2017).
Wang, C. et al. In situ formed reactive oxygen species-responsive scaffold with gemcitabine and checkpoint inhibitor for combination therapy. Sci. Transl. Med. 10, eaan3682 (2018).
Wang, C., Wen, D. & Gu, Z. Cellular Bioparticulates with Therapeutics for Cancer Immunotherapy. Bioconjugate Chem. 29, 702-708 (2017).
Wang, C., Ye, Y., Hu, Q., Bellotti, A. & Gu, Z. Tailoring biomaterials for cancer immunotherapy: Emerging trends and future outlook. Adv. Mater. 29, 1606036 (2017).
Wang, Y.-C. et al. Notch signaling determines the M1 versus M2 polarization of macrophages in antitumor immune responses. Cancer Res. 70, 4840-4849 (2010).
Zhao, Y. et al. A Preloaded Amorphous Calcium Carbonate/Doxorubicin@ Silica Nanoreactor for pH-Responsive Delivery of an Anticancer Drug. Angew. Chem. Int. Ed. Engl. 54, 919-922 (2015).
Zou, W., Wolchok, J.D. & Chen, L. PD-L1 (B7-H1) and PD-1 pathway blockade for cancer therapy: Mechanisms, response biomarkers, and combinations. Sci. Transl. Med. 8, 328rv324 (2016).

## Claims

1. An immunotherapeutic bioresponsive gel matrix comprising a first component and a second component, wherein the first component comprises fibrinogen, wherein gelation of the matrix occurs when the first component comes into contact with the second component; and wherein at least one the components comprises at least one first nanoparticle; wherein the at least one first nanoparticle comprises a pH responsive material and an anti-CD47 antibody; and/or preferably wherein the pH responsive material comprises CaCOs, chitosan, hyaluronic acid, dextran, and/or polymers of dextran monomers; and/or wherein the second component comprises thrombin.

2. The immunotherapeutic bioresponsive gel matrix of claim 1, wherein the immunotherapeutic bioresponsive gel matrix further comprises at least one second nanoparticle.

3. The immunotherapeutic bioresponsive gel matrix of claim 1 or 2, wherein the first component comprises the at least one first nanoparticle.

4. The immunotherapeutic bioresponsive gel matrix of claim 3, wherein the first component further comprises a second anti-cancer agent, blockade inhibitor, or immunomodulatory agent.

5. The immunotherapeutic bioresponsive gel matrix of claim 3, wherein the second component comprises a second anti-cancer agent, blockade inhibitor, or immunomodulatory agent.

6. The immunotherapeutic bioresponsive gel matrix of any of claims 1 or 2, wherein the second component comprises the at least one first nanoparticle.

7. The immunotherapeutic bioresponsive gel matrix of claim 6, wherein the second component further comprises a second anti-cancer agent, blockade inhibitor, or immunomodulatory agent.

8. The immunotherapeutic bioresponsive gel matrix of claim 6, wherein the first component comprises a second anti-cancer agent, blockade inhibitor, or immunomodulatory agent.

9. The immunotherapeutic bioresponsive gel matrix of any of claims 1-8, wherein less than 30% anti-cancer agent, blockade inhibitor, or immunomodulatory agent is released 140 hours following administration of the matrix.

10. The immunotherapeutic bioresponsive gel matrixes of any of claims 1-9 for use in a method of inhibiting metastasis at the site of surgical resection of a cancer tumor comprising administering to the site of the resection .

11. The matrix of claim 10 for use in a method of inhibiting metastasis at the site of surgical resection of a cancer tumor, wherein the first component and second component are delivered to the subject separately optionally, wherein the first component is delivered to the site of the tumor in the subject before the second component; optionally wherein the first component is delivered to the site of the tumor in the subject after the second component.

12. The matrix of claim 10 or 11 for use in a method of inhibiting metastasis at the site of surgical resection of a cancer tumor, wherein the first component is administered at least 1, 2, 3, 4, 5, 10, 15, 20, 25, 30, 35, 40, 45, 50, 55 minutes, 1, 2, 3, 4, 5, 6, 7, 8, 9, 10, 11, 12, 18, 24, 36, 48, or 72 hours after the second component.

13. The matrix of claim 10 for use in a method of inhibiting metastasis at the site of surgical resection of a cancer tumor, wherein the first and second components are mixed together prior to administration to the subject.

14. The matrix of any of claims 10-13 for use in a method of inhibiting metastasis at the site of surgical resection of a cancer tumor, wherein at least one of the first and second component is administered to the site of resection prior to the removal of the tumor.

15. An immunotherapeutic bioresponsive gel matrix comprising a first component and a second component, wherein the first component comprises fibrinogen, wherein gelation of the matrix occurs when the first component comes into contact with the second component; and wherein at least one of the components comprises at least one first nanoparticle; wherein the at least one first nanoparticle comprises a pH responsive material and an anti-CD47 antibody; preferably wherein the pH responsive material comprises CaCO3, chitosan, hyaluronic acid, dextran, and/or polymers of dextran monomers for use in a method of increasing the innate or adaptive immune response to a tumor following resection of the tumor comprising administering to a subject with a tumor.

## Patentansprüche

1. Eine immuntherapeutische bioresponsive Gelmatrix, welche eine erste Komponente und eine zweite Komponente umfasst, wobei die erste Komponente Fibrinogen umfasst, wobei die Gelierung der Matrix auftritt, wenn die erste Komponente mit der zweiten Komponente in Kontakt kommt; und wobei mindestens eine der Komponenten mindestens ein erstes Nanopartikel umfasst; wobei der mindestens eine erste Nanopartikel ein auf den pH-Wert reagierendes Material und einen Anti-CD47-Antikörper umfasst; und/oder vorzugsweise wobei das auf den pH-Wert reagierende Material CaCO₃, Chitosan, Hyaluronsäure, Dextran und/oder Polymere von Dextranmonomeren umfasst; und/oder wobei die zweite Komponente Thrombin umfasst.

2. Die immuntherapeutische bioresponsive Gelmatrix nach Anspruch 1, wobei die immuntherapeutische bioresponsive Gelmatrix ferner mindestens ein zweites Nanopartikel umfasst.

3. Die immuntherapeutische bioresponsive Gelmatrix nach Anspruch 1 oder 2, wobei die erste Komponente das mindestens eine erste Nanopartikel umfasst.

4. Die immuntherapeutische bioresponsive Gelmatrix nach Anspruch 3, wobei die erste Komponente außerdem ein zweites Anti-Krebsmittel, einen Blockade-Inhibitor oder ein immunmodulatorisches Mittel umfasst.

5. Die immuntherapeutische bioresponsive Gelmatrix nach Anspruch 3, wobei die zweite Komponente ein zweites Anti-Krebsmittel, einen Blockade-Inhibitor oder ein immunmodulatorisches Mittel umfasst.

6. Die immuntherapeutische bioresponsive Gelmatrix nach einem der Ansprüche 1 oder 2, wobei die zweite Komponente das mindestens eine erste Nanopartikel umfasst.

7. Die immuntherapeutische bioresponsive Gelmatrix nach Anspruch 6, wobei die zweite Komponente ferner ein zweites Anti-Krebsmittel, einen Blockade-Inhibitor oder ein immunmodulatorisches Mittel umfasst.

8. Die immuntherapeutische bioresponsive Gelmatrix nach Anspruch 6, wobei die erste Komponente ein zweites Anti-Krebsmittel, einen Blockade-Inhibitor oder ein immunmodulatorisches Mittel umfasst.

9. Die immuntherapeutische bioresponsive Gelmatrix nach einem der Ansprüche 1-8, wobei weniger als 30% des Antikrebsmittels, Blockade-Inhibitors oder immunmodulatorischen Mittels 140 Stunden nach Verabreichung der Matrix freigesetzt werden.

10. Die immuntherapeutische bioresponsive Gelmatrix nach einem der Ansprüche 1-9 zur Verwendung in einem Verfahren zur Hemmung der Metastasierung an der Stelle der chirurgischen Resektion eines Krebstumors, umfassend die Verabreichung an die Stelle der Resektion.

11. Die Matrix nach Anspruch 10 zur Verwendung in einem Verfahren zur Hemmung der Metastasierung an der Stelle der chirurgischen Resektion eines Krebstumors, wobei die erste Komponente und die zweite Komponente dem Subjekt getrennt verabreicht werden, optional, wobei die erste Komponente vor der zweiten Komponente an die Stelle des Tumors in dem Subjekt verabreicht wird; optional, wobei die erste Komponente nach der zweiten Komponente an die Stelle des Tumors in dem Subjekt verabreicht wird.

12. Die Matrix nach Anspruch 10 oder 11 zur Verwendung in einem Verfahren zur Hemmung der Metastasierung an der Stelle der chirurgischen Resektion eines Krebstumors, wobei die erste Komponente mindestens 1, 2, 3, 4, 5, 10, 15, 20, 25, 30, 35, 40, 45, 50, 55 Minuten, 1, 2, 3, 4, 5, 6, 7, 8, 9, 10, 11, 12, 18, 24, 36, 48, oder 72 Stunden nach der zweiten Komponente verabreicht wird.

13. Die Matrix nach Anspruch 10 zur Verwendung in einem Verfahren zur Hemmung der Metastasierung an der Stelle der chirurgischen Resektion eines Krebstumors, wobei die erste und die zweite Komponente vor der Verabreichung an das Subjekt miteinander vermischt werden.

14. Die Matrix nach einem der Ansprüche 10-13 zur Verwendung in einem Verfahren zur Hemmung der Metastasierung an der Stelle der chirurgischen Resektion eines Krebstumors, wobei mindestens eine der ersten und zweiten Komponente vor der Entfernung des Tumors an die Resektionsstelle verabreicht wird.

15. Eine immuntherapeutische bioresponsive Gelmatrix, welche eine erste Komponente und eine zweite Komponente umfasst, wobei die erste Komponente Fibrinogen umfasst, wobei die Gelierung der Matrix auftritt, wenn die erste Komponente mit der zweiten Komponente in Kontakt kommt; und wobei mindestens eine der Komponenten mindestens ein erstes Nanopartikel umfasst; wobei der mindestens eine erste Nanopartikel ein auf den pH-Wert reagierendes Material und einen Anti-CD47-Antikörper umfasst; und/oder vorzugsweise wobei das auf den pH-Wert reagierende Material CaCO3, Chitosan, Hyaluronsäure, Dextran und/oder Polymere von Dextranmonomeren umfasst, zur Verwendung in einem Verfahren zur Erhöhung der angeborenen oder adaptiven Immunantwort auf einen Tumor nach Resektion des Tumors, umfassend die Verabreichung an ein Subjekt mit einem Tumor.

## Revendications

1. Matrice de gel biosensible immunothérapeutique comprenant un premier composant et un second composant, dans laquelle le premier composant comprend du fibrinogène, dans laquelle la gélification de la matrice se produit lorsque le premier composant entre en contact avec le second composant ; et dans laquelle au moins l'un des composants comprend au moins une première nanoparticule ; dans laquelle l'au moins une première nanoparticule comprend un matériau sensible au pH et un anticorps anti-CD47 ; et/ou de préférence, dans laquelle le matériau sensible au pH comprend du CaCO₃, du chitosane, de l'acide hyaluronique, du dextrane et/ou des polymères de monomères dextrane ; et/ou dans laquelle le second composant comprend de la thrombine.

2. Matrice de gel biosensible immunothérapeutique selon la revendication 1, dans laquelle la matrice de gel biosensible immunothérapeutique comprend en outre au moins une seconde nanoparticule.

3. Matrice de gel biosensible immunothérapeutique selon la revendication 1 ou 2, dans laquelle le premier composant comprend l'au moins une première nanoparticule.

4. Matrice de gel biosensible immunothérapeutique selon la revendication 3, dans laquelle le premier composant comprend en outre un second agent anticancéreux, un inhibiteur de blocage ou un agent immunomodulateur.

5. Matrice de gel biosensible immunothérapeutique selon la revendication 3, dans laquelle le second composant comprend un second agent anticancéreux, un inhibiteur de blocage ou un agent immunomodulateur.

6. Matrice de gel biosensible immunothérapeutique selon l'une quelconque des revendications 1 ou 2, dans laquelle le second composant comprend l'au moins une première nanoparticule.

7. Matrice de gel biosensible immunothérapeutique selon la revendication 6, dans laquelle le second composant comprend en outre un second agent anticancéreux, un inhibiteur de blocage ou un agent immunomodulateur.

8. Matrice de gel biosensible immunothérapeutique selon la revendication 6, dans laquelle le premier composant comprend un second agent anticancéreux, un inhibiteur de blocage ou un agent immunomodulateur.

9. Matrice de gel biosensible immunothérapeutique selon l'une quelconque des revendications 1 à 8, dans laquelle moins de 30 % d'un agent anticancéreux, d'un inhibiteur de blocage ou d'un agent immunomodulateur sont libérés 140 heures après l'administration de la matrice.

10. Matrices de gel biosensibles immunothérapeutiques selon l'une quelconque des revendications 1 à 9, destinées à être utilisées dans un procédé d'inhibition des métastases au niveau du site de résection chirurgicale d'une tumeur cancéreuse comprenant l'administration au site de résection.

11. Matrice selon la revendication 10, destinée à être utilisée dans une méthode d'inhibition des métastases au niveau du le site de résection chirurgicale d'une tumeur cancéreuse, dans laquelle le premier composant et le second composant sont délivrés au sujet séparément éventuellement, dans laquelle le premier composant est délivré au site de la tumeur chez le sujet avant le second composant ; éventuellement, dans laquelle le premier composant est délivré au site de la tumeur chez le sujet après le second composant.

12. Matrice selon la revendication 10 ou 11, destinée à être utilisée dans un procédé d'inhibition des métastases au niveau du site de résection chirurgicale d'une tumeur cancéreuse, dans laquelle le premier composant est administré au moins 1, 2, 3, 4, 5, 10, 15, 20, 25, 30, 35, 40, 45, 50, 55 minutes, 1, 2, 3, 4, 5, 6, 7, 8, 9, 10, 11, 12, 18, 24, 36, 48 ou 72 heures après le second composant.

13. Matrice selon la revendication 10, destinée à être utilisée dans un procédé d'inhibition des métastases au niveau du site de résection chirurgicale d'une tumeur cancéreuse, dans laquelle les premier et second composants sont mélangés ensemble avant l'administration au sujet.

14. Matrice selon l'une quelconque des revendications 10 à 13, destinée à être utilisée dans un procédé d'inhibition des métastases au niveau du site de résection chirurgicale d'une tumeur cancéreuse, dans laquelle au moins l'un des premier et second composants est administré au site de résection avant le retrait de la tumeur.

15. Matrice de gel biosensible immunothérapeutique comprenant un premier composant et un second composant, dans laquelle le premier composant comprend du fibrinogène, dans laquelle la gélification de la matrice se produit lorsque le premier composant entre en contact avec le second composant ; et dans laquelle l'au moins un des composants comprend au moins une première nanoparticule ; dans laquelle au moins une première nanoparticule comprend au moins un matériau sensible au pH et un anticorps anti-CD47 ; de préférence, dans laquelle le matériau sensible au pH comprend du CaCO3, du chitosane, de l'acide hyaluronique, du dextrane et/ou des polymères de monomères dextrane, destinée à être utilisée dans un procédé permettant d'augmenter la réponse immunitaire innée ou adaptative à une tumeur après la résection de la tumeur, comprenant l'administration à un sujet avec une tumeur.
